# EUROPEAN PATENT SPECIFICATION

(11) **EP 3 677 694 B1**
(45) Date of publication and mention of the grant of the patent: **20.07.2022**
(21) Application number: 19210010.5
(22) Date of filing: 19.09.2014
(51) Int. Cl.: C12Q 1/6886

(54) **COMPOSITIONS AND METHODS FOR THE ANALYSIS OF RADIOSENSITIVITY**
ZUSAMMENSETZUNGEN UND VERFAHREN ZUR ANALYSE VON STRAHLENEMPFINDLICHKEIT
COMPOSITIONS ET PROCÉDÉS D'ANALYSE DE LA RADIOSENSIBILITÉ

(30) Priority: 20.09.2013 US 201361880717 P
(43) Date of publication of application: 08.07.2020
(62) Divisional of application: 14845384.8
(73) Proprietor: The Regents of The University of Michigan, Ann Arbor, Michigan 48109-2590 (US)
(72) Inventor: SPEERS, Corey, Hamburg Twp., MI 48189 (US); FENG, Felix, Ann Arbor, MI 48109-5010 (US); PIERCE, Lori, Ann Arbor, MI 48109-5936 (US); ZHAO, Shuang, Ann Arbor, MI 48105 (US)
(74) Representative: Glawe, Delfs, Moll

(56) References cited:
- WO-A2-2009/036388
- WO-A2-2013/132354
- S. A. ESCHRICH ET AL: "Validation of a Radiosensitivity Molecular Signature in Breast Cancer", CLINICAL CANCER RESEARCH, vol. 18, no. 18, 25 July 2012 (2012-07-25) , pages 5134-5143, XP055352422, US ISSN: 1078-0432, DOI: 10.1158/1078-0432.CCR-12-0891
- HAN SANG KIM ET AL: "Identification of a radiosensitivity signature using integrative metaanalysis of published microarray data for NCI-60 cancer cells", BMC GENOMICS, BIOMED CENTRAL LTD, LONDON, UK, vol. 13, no. 1, 30 July 2012 (2012-07-30), page 348, XP021106691, ISSN: 1471-2164, DOI: 10.1186/1471-2164-13-348

## Description

### CROSS-REFERENCE TO RELATED APPLICATION

The present application claims priority to U.S. Provisional Patent Application Serial No. 61/880,717, filed September 20, 2013.

### FIELD

Provided herein are compositions and methods for the analysis of radiosensitivity, to, for example, assess the efficacy or select therapeutic agents for the treatment and/or diagnosis of cancer.

### BACKGROUND

Multiple randomized clinical trials have demonstrated the benefit of adjuvant radiation therapy after breast conserving surgery. However, it is clear that current adjuvant radiotherapy approaches result in overtreatment of many patients who are unlikely to recur after surgery alone. Conversely, there are subsets of patients who, despite standard multimodality treatment including radiation, will develop local recurrence. Thus, there is a clear need to identify these two populations: those who are currently over-treated, and those who need further treatment intensification.

A kit comprising reagents for the specific quantification of gene expression levels or two or more signature genes in a cell is known from WO 2009/036388 A2. However, said kit is different from the kit as defined in the enclosed claim set comprising reagents for analyzing the expression of the 51 genes of Table 6 and no other markers, which in turn provides for an improved gene signature for radiosensitivity of a cell and is not derivable from said prior art. WO 2013/132354 A2 discloses a method of selecting a subject with breast cancer for radiotherapy after mastectomy. However, it uses a different gene signature from the one as defined in the enclosed claim set.

### SUMMARY

The invention is set out in the appended claims. The embodiments of the description which do not fall within the scope of said claims are provided for illustrative purposes only and do not form part of the present invention. Provided herein are compositions and methods for the analysis of radiosensitivity, to, for example, assess the efficacy or select therapeutic agents for the treatment and/or diagnosis of cancer.

Provided herein are compositions, methods, or systems that assess the expression of two or more markers described in Table 1, 2, 5 or 6 for the selection of therapy for a subject.

The present description provides methods for assessing the radiosensitivity of a cell comprising detecting expression of a plurality genes indicative of radiosensitivity and/or radioresistance. The genes indicative of radiosensitivity and/or radioresistance are the genes from Table 6. Gene expression may be detected by measuring mRNA levels. Gene expression may be detected by measuring protein levels. The expression of plurality genes indicative of radiosensitivity and/or radioresistance may be differentially weighted to determine the radiosensitivity of a cell. The cell can be a breast cancer cell.

The present description provides methods of determining a treatment course for a subject suffering from breast cancer comprising: (a) assessing the radiosensitivity/ radioresistance of the cancer using methods/kits described herein; and (b) selecting a suitable treatment course based on the radiosensitivity/radioresistance of the cancer.

The present invention provides a kit as defined in the claims. The description further discloses kits for assessing radiosensitivity comprising reagents for detecting expression of a plurality genes indicative of radiosensitivity and/or radioresistance, including that, the genes indicative of radiosensitivity are selected from Table 1, that the genes indicative of radioresistance are selected from Table 2, that the genes indicative of radiosensitivity and/or radioresistance are selected from Table 5, or that the genes indicative of radiosensitivity and/or radioresistance are selected from Table 6. In some embodiments, reagents are selected from antibodies, aptamers, nucleic acid probes, and primers.

The description further discloses methods for assessing the radiosensitivity of a population of cells within a subject, comprising: (a) receiving a sample obtained from the population of cells; (b) quantitating the level of expression of a one or more genes indicative or radiosensitivity in said sample; and (c) quantitating the level of expression of a one or more genes indicative or radioresistance in said sample. In some embodiments, methods further provide (d) using a computer-based analysis program is used to convert the data generated in steps (b) and (c) into an radiosensitivity assessment for the population of cells from which the sample was obtained. In some embodiments, methods further provide (e) generating a report characterizing the sample as having been obtained from a radiosensitivity of a population of cells within a subject.

The description further discloses methods for assessing the radiosensitivity of a population of cells within a subject, comprising: (a) obtaining a sample obtained from the population of cells; (b) having the sample analyzed to quantitate the level of expression of a one or more genes indicative or radiosensitivity in said sample; (c) having the sample analyzed to quantitate the level of expression of a one or more genes indicative or radioresistance in said sample; and (d) receiving a report related to the radiosensitivity of the population of cells. In some embodiments, said report is generated using a computer-based analysis program is used to convert the data generated in steps (b) and (c) into a radiosensitivity assessment for the population of cells from which the sample was obtained. In some said report characterizes the sample as having been obtained from a radiosensitivity population of cells within a subject.

In some embodiments, provided herein are kits as defined in the claims. The description further discloses kits for conducting assays to identify the expression of the markers. The kits may comprise reagents (e.g., probes, primers, buffers, etc.) and other components (e.g., software, instructions, data sets) necessary, sufficient, or useful for conducting any of the methods described herein. The kits may provide reagents in multiplex format for the simultaneous analysis of multiple markers (e.g., on one reaction mixture, container, or devices (e.g., multi-well card or plate)). The kits may comprise, may consist, or may consist essentially of the reagents needed to assess the plurality of markers to provide a desired diagnostic result. For example, for costefficiency, such kits do not include reagents (e.g., primers and probes) for analyzing other markers (e.g., rather than using a gene chip to assess expression of all expression markers, the kit only detects the specific markers needed to make the diagnostic assessment).

Also provided herein are reaction mixtures comprising sample nucleic acid or proteins and reagents (e.g., from any of the above kits or methods) for assessing expression of a marker from Table 1, Table 2, Table 5, and/or Table 6. The reaction mixtures may be generated by conducting a method as described herein.

A software or hardware component may receive the results of multiple assays and/or markers and determines a single value result to report to a user that indicates a conclusion (e.g., cancer risk, drug efficacy, prognosis, etc.). Related embodiments calculate a risk factor based on a mathematical combination (e.g., a weighted combination, a linear combination) of the results from multiple assays and/or markers.

A storage medium and memory components may be comprised. Memory components (e.g., volatile and/or nonvolatile memory) find use in storing instructions (e.g., an embodiment of a process as provided herein) and/or data (e.g., a work piece such as methylation measurements, sequences, and statistical descriptions associated therewith). The present description also relates to systems also comprising one or more of a CPU, a graphics card, and a user interface (e.g., comprising an output device such as display and an input device such as a keyboard).

Programmable machines associated with the technology comprise conventional extant technologies and technologies in development or yet to be developed (e.g., a quantum computer, a chemical computer, a DNA computer, an optical computer, a spintronics based computer, etc.).

The technology may comprise a wired (e.g., metallic cable, fiber optic) or wireless transmission medium for transmitting data. For example, data transmission over a network (e.g., a local area network (LAN), a wide area network (WAN), an ad-hoc network, the internet, etc.) or programmable machines are present on such a network as peers and the programmable machines may have a client/server relationship.

Data may be stored on a computer-readable storage medium such as a hard disk, flash memory, optical media, a floppy disk, etc.

The technology provided herein may be associated with a plurality of programmable devices that operate in concert to perform a method as described herein. For example, a plurality of computers (e.g., connected by a network) may work in parallel to collect and process data, e.g., in an implementation of cluster computing or grid computing or some other distributed computer architecture that relies on complete computers (with onboard CPUs, storage, power supplies, network interfaces, etc.) connected to a network (private, public, or the internet) by a conventional network interface, such as Ethernet, fiber optic, or by a wireless network technology.

For example a computer that includes a computer-readable medium may be provided. This includes a random access memory (RAM) coupled to a processor. The processor executes computer-executable program instructions stored in memory. Such processors may include a microprocessor, an ASIC, a state machine, or other processor, and can be any of a number of computer processors, such as processors from Intel Corporation of Santa Clara, California and Motorola Corporation of Schaumburg, Illinois. Such processors include, or may be in communication with, media, for example computer-readable media, which stores instructions that, when executed by the processor, cause the processor to perform the steps described herein.

Examples of computer-readable media include, but are not limited to, an electronic, optical, magnetic, or other storage or transmission device capable of providing a processor with computer-readable instructions. Other examples of suitable media include, but are not limited to, a floppy disk, CD-ROM, DVD, magnetic disk, memory chip, ROM, RAM, an ASIC, a configured processor, all optical media, all magnetic tape or other magnetic media, or any other medium from which a computer processor can read instructions. Also, various other forms of computer-readable media may transmit or carry instructions to a computer, including a router, private or public network, or other transmission device or channel, both wired and wireless. The instructions may comprise code from any suitable computer-programming language, including, for example, C, C++, C#, Visual Basic, Java, Python, Perl, and JavaScript.

Computers are connected in some embodiments to a network. Computers may also include a number of external or internal devices such as a mouse, a CD-ROM, DVD, a keyboard, a display, or other input or output devices. Examples of computers are personal computers, digital assistants, personal digital assistants, cellular phones, mobile phones, smart phones, pagers, digital tablets, laptop computers, internet appliances, and other processor-based devices. In general, the computers related to aspects of the technology provided herein may be any type of processor-based platform that operates on any operating system, such as Microsoft Windows, Linux, UNIX, Mac OS X, etc., capable of supporting one or more programs comprising the technology provided herein. A personal computer executing other application programs (e.g., applications) may be comprised. The applications can be contained in memory and can include, for example, a word processing application, a spreadsheet application, an email application, an instant messenger application, a presentation application, an Internet browser application, a calendar/organizer application, and any other application capable of being executed by a client device.

All such components, computers, and systems described herein as associated with the technology may be logical or virtual.

### BRIEF DESCRIPTION OF DRAWINGS

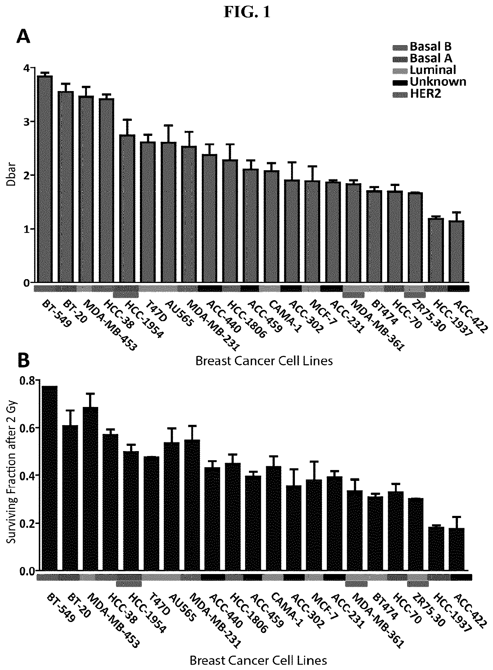

Figure 1 shows clonogenic survival assays assessing the baseline radiation sensitivity in a panel of 21 breast cancer cell lines. Clonogenic survival assessing Dbar (A) and surviving fraction after 2 Gy (B) identifies a range of radiation sensitivity in human breast cancer cell lines (SF 77%-17%) with no significant correlation to the intrinsic breast cancer subtypes. Grouping of breast cancer cell lines into resistant, moderately resistant, and radiation sensitive cell lines shows a mixture of each intrinsic subtype in these categories.

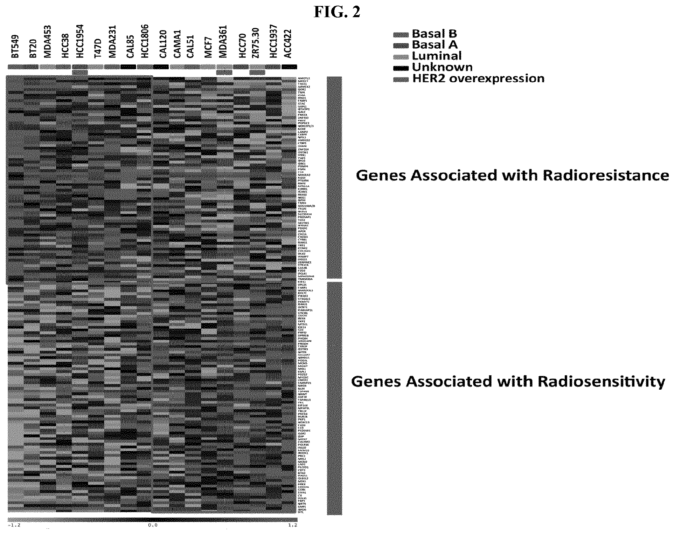

Figure 2 depicts unsupervised clustering demonstrating that the radiation response phenotype is independent to and not correlated with the intrinsic subtypes of human breast cancer.

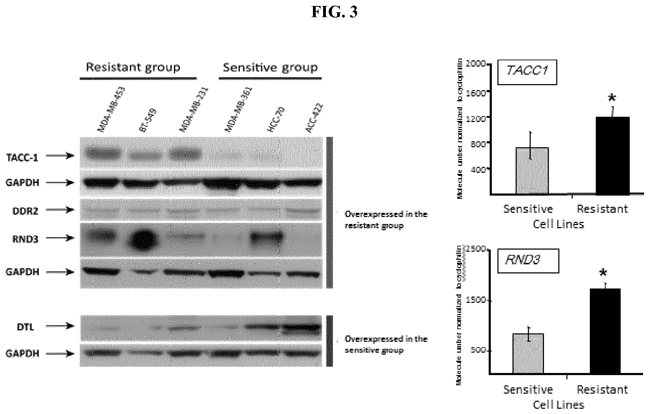

Figure 3 shows validation of gene overexpression in breast cancer cell lines by assessing protein and RNA expression.

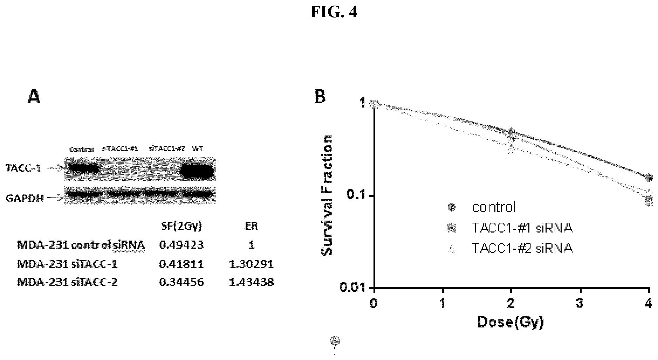

Figure 4 shows clonogenic survival assays of several genes identified as being overexpressed in the radioresistant signature are involved in radioresistance.

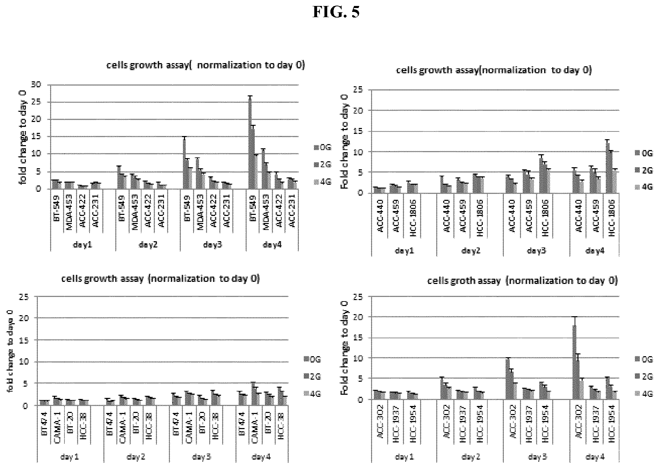

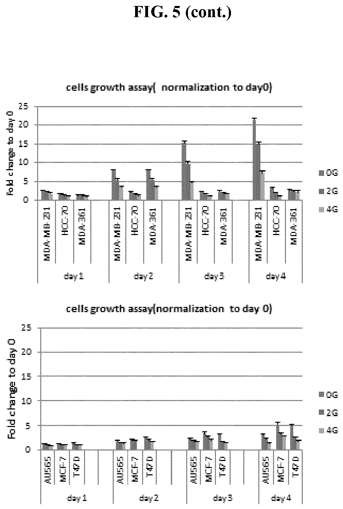

Figure 5 shows graphs depicting cells growth assays for breast cancer cell lines treated with radiation.

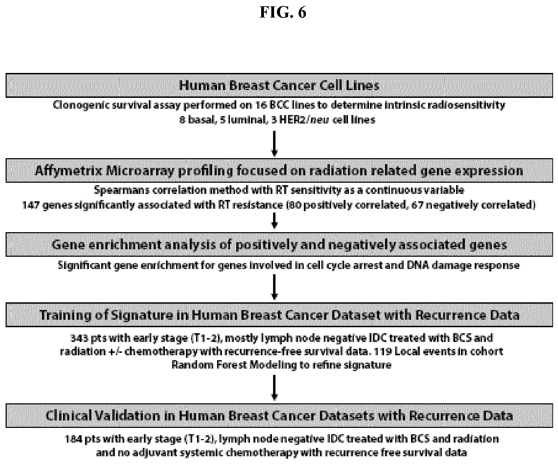

Figure 6 shows an overview of the radiation response signature development strategy utilizing intrinsic radiation sensitivity of human breast cancer cell lines.

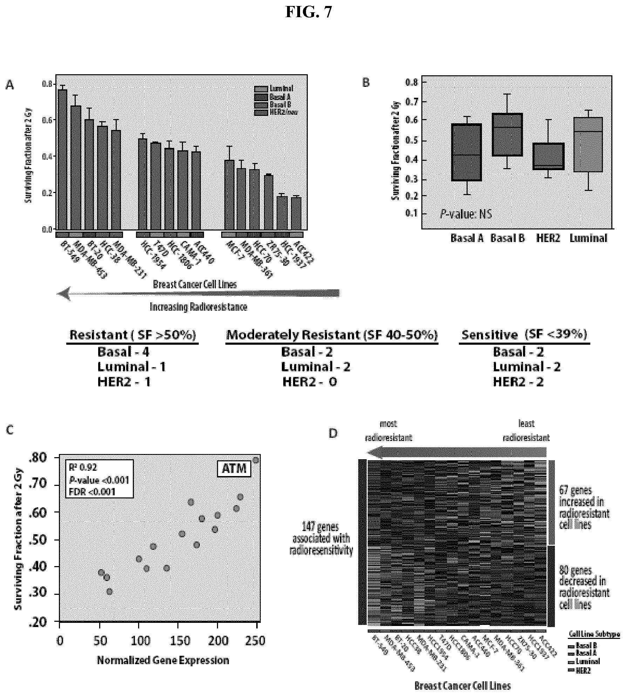

Figure 7A-D shows results relating to clonogenic survival assays performed to assess the baseline radiation sensitivity in a panel of 16 breast cancer cell lines. Clonogenic survival assessing the surviving fraction of cells after exposure to a standard fraction of radiation (SF-2Gy) (A) identifies a range of radiation sensitivity in human breast cancer cell lines (SF-2 Gy 77%-17%). Box and whisper plot depicting minimum and maximum SF-2Gy values. ANOVA testing shows no significant difference in radiosensitivity between the intrinsic breast cancer subtypes (B). Grouping of breast cancer cell lines into resistant, moderately resistant, and radiation sensitive cell lines shows a mixture of each intrinsic subtype in these categories. The radiation sensitivity signature was generated by selecting genes whose expression was significantly correlated with intrinsic radiation sensitivity (SF-2Gy) with a 2 fold difference in expression with representative correlation scatter plot depicted for the gene ATM (C). Based on these selection criteria, 147 genes were identified as being correlated with radiation sensitivity, with the expression level of 80 genes correlated with radiation resistance and 67 genes negatively correlated with radiation resistance. A heatmap depicting relative gene expression of these 147 genes ordered by the intrinsic radiosensitivity of the 16 breast cancer cell lines is depicted (D).

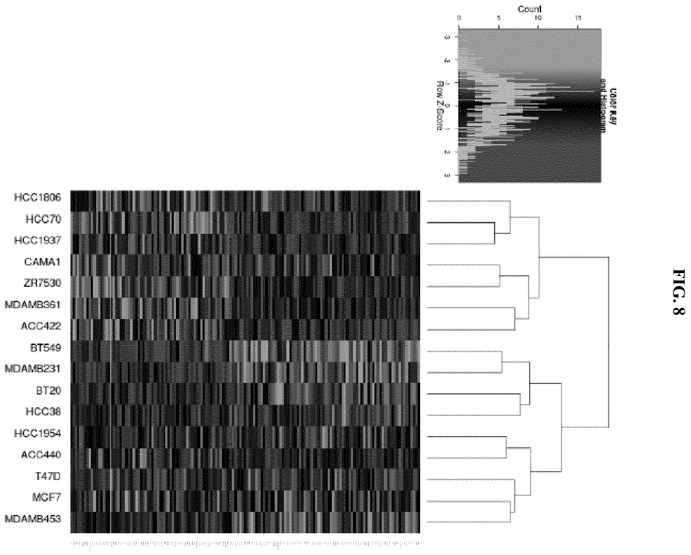

Figure 8 shows unsupervised hierarchical clustering using the gene expression of the 147 differentially expressed genes accurately clusters the radiation resistant cell lines (SF2 Gy >45%) from the radiation sensitive cell lines (SF 2 Gy <45%).

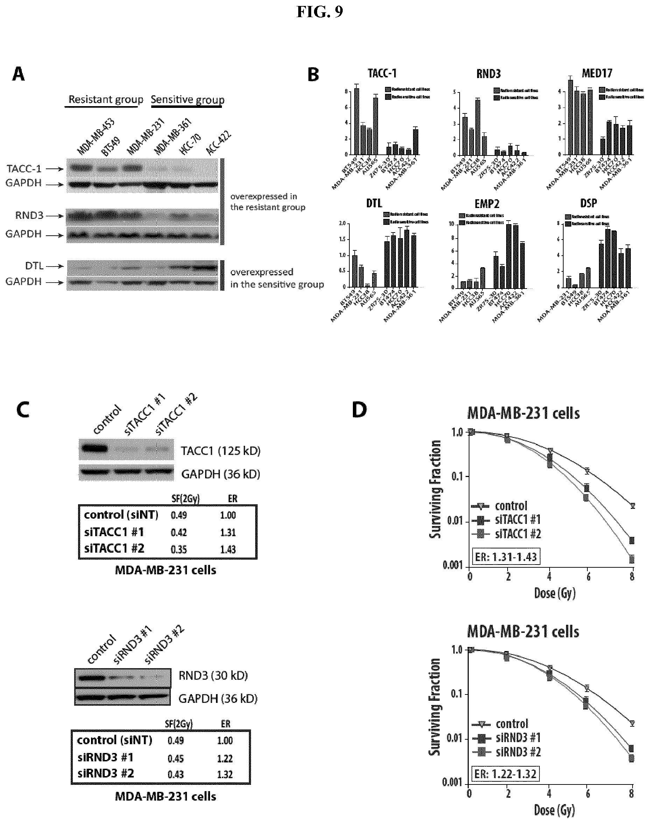

Figure 9A-D shows validation of gene overexpression in breast cancer cell lines by assessing protein and RNA expression. The expression of selected genes identified in the array profiling were validated as being more highly expressed in the radiation resistant cell lines compared to the radiation sensitive cell lines by western blotting (A). Expression data for 6 representative genes (TACC1, DDR2, RND3, DTL, ATM, RAD51) from a panel or radiation resistant and radiation sensitive breast cancer cell lines are shown (B). Data are represented as mean ± SEM. Clonogenic survival assays validates several genes identified as being overexpressed in the radioresistant signature are involved in radioresistance. Knockdown experiments with siRNA designed against TACC1, a gene identified in the radioresistance signature, effectively knocks down the expression of TACC1 (C). Clonogenic survival assays in MDA-MB-231 breast cancer cells after siRNA knockdown of TACC1 show significant sensitization of these cells to ionizing radiation with an enhancement ratio of 1.1-1.4 (D).

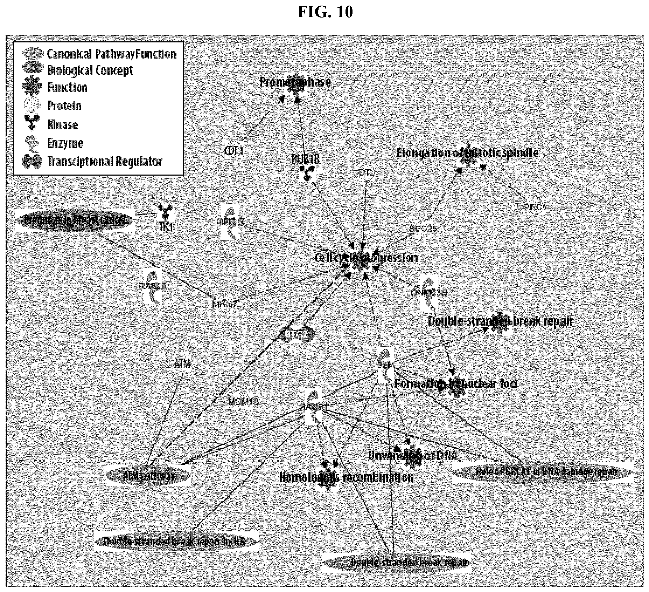

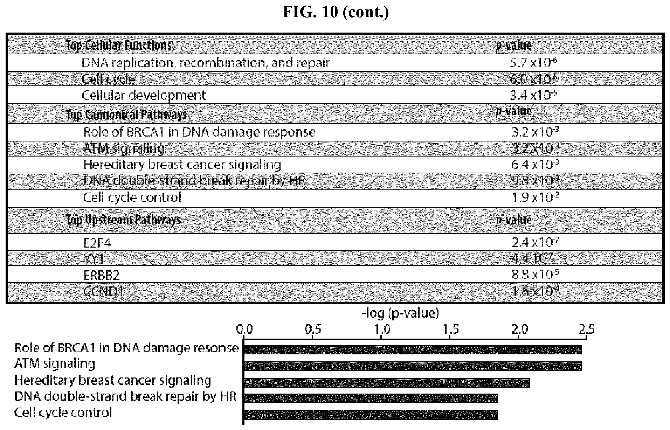

Figure 10 shows gene set enrichment and gene ontology enrichment analysis demonstrates that the radiation sensitivity signature is significantly enriched for concepts related to radiation response including DNA repair, cell cycle, and DNA damage response.

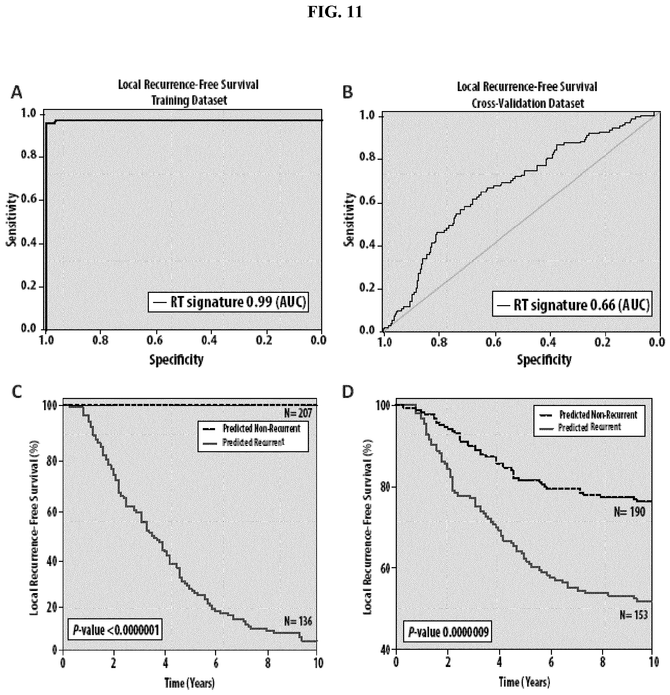

Figure 11A-D shows receiver operating characteristic (ROC) curves and Kaplan-Meier survival analysis in training and cross-validation dataset with univariable and multivariable analysis. AUC values from the training set show perfect performance (A) and Kaplan-Meier analysis demonstrates complete separation of the curves between those predicted to recur and not predicted to recur (B). Univariable (C) and multivariable (D) analysis identifies the radiation signature score as the variable most strongly associated with local recurrence.

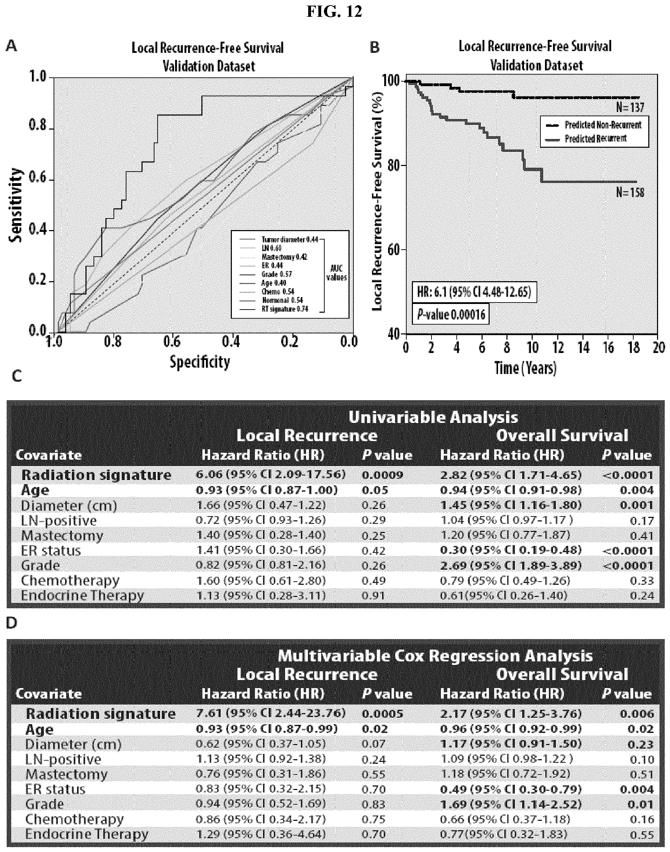

Figure 12A-D shows receiver operating characteristic (ROC) curves and Kaplan-Meier survival estimate analysis in validation dataset with univariable and multivariable analysis. AUC values from the validation dataset show the radiation signature score outperforms every other clinical and pathologic parameter (A). Kaplan-Meier survival estimate analysis and hazard ratios are depicted in panel B. Univariable (C) and multivariable (D) analysis identifies the radiation signature score as the variable most strongly associated with local recurrence and overall survival.

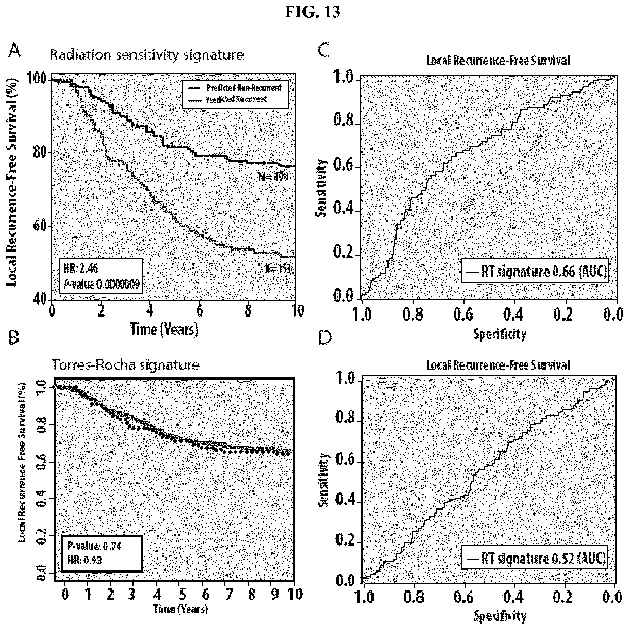

Figure 13A-D shows performance of the radiation sensitivity signature with the previously published radiation sensitivity signature in the training dataset. Kaplan-Meier local recurrence survival estimates in our signature (A) and the previously published signature (B). ROC curves between the radiation sensitivity signature (C) and the previously published signature (D).

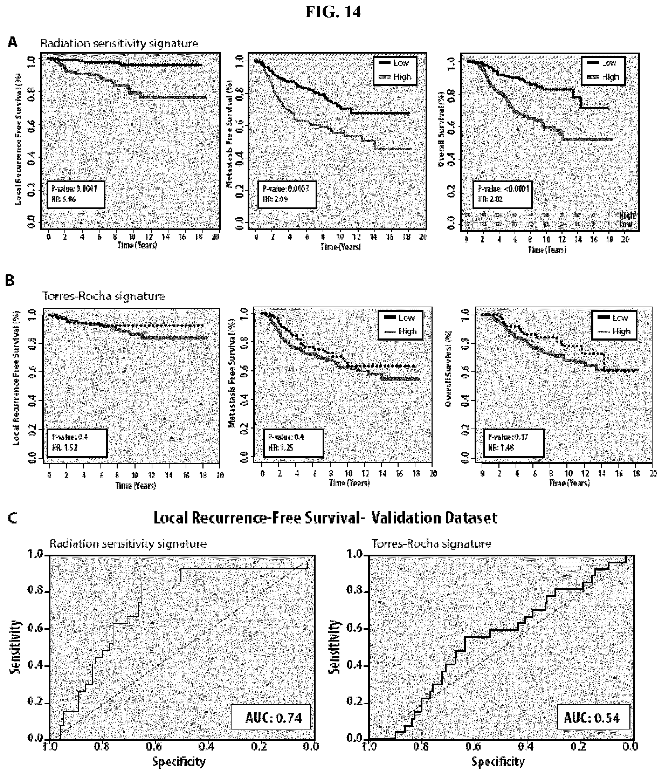

Figure 14A-C shows performance of the radiation sensitivity signature with the previously published radiation sensitivity signature in the validations dataset. Kaplan-Meier local recurrence, metastasis-free, and overall survival estimates in the radiation sensitivity signature (A) and the previously published signature (B). ROC curves predicting local recurrence between the radiation sensitivity signature and the previously published signature (C).

### DETAILED DESCRIPTION

The invention is set out in the appended claims. The embodiments of the description which do not fall within the scope of said claims are provided for illustrative purposes only and do not form part of the present invention. Provided herein are compositions and methods for the analysis of radiosensitivity, to, for example, assess the efficacy or select therapeutic agents for the treatment and/or diagnosis of cancer.

In this detailed description, for purposes of explanation, numerous specific details are set forth to provide a thorough understanding of the invention as defined in the claims. One skilled in the art will appreciate, however, that these various embodiments may be practiced with or without these specific details. In other instances, structures and devices are shown in block diagram form. Furthermore, one skilled in the art can readily appreciate that the specific sequences in which methods are presented and performed are illustrative.

Unless defined otherwise, all technical and scientific terms used herein have the same meaning as is commonly understood by one of ordinary skill in the art to which the various embodiments described herein belongs. When definitions of terms in incorporated references appear to differ from the definitions provided in the present teachings, the definition provided in the present teachings shall control.

### Definitions

To facilitate an understanding of the present technology, a number of terms and phrases are defined below. Additional definitions are set forth throughout the detailed description.

Throughout the specification and claims, the following terms take the meanings explicitly associated herein, unless the context clearly dictates otherwise. The phrase "in one embodiment" as used herein does not necessarily refer to the same embodiment, though it may. Furthermore, the phrase "in another embodiment" as used herein does not necessarily refer to a different embodiment, although it may. Thus, as described below, various embodiments of the invention may be readily combined, without departing from the scope or spirit of the invention.

In addition, as used herein, the term "or" is an inclusive "or" operator and is equivalent to the term "and/or" unless the context clearly dictates otherwise. The term "based on" is not exclusive and allows for being based on additional factors not described, unless the context clearly dictates otherwise. In addition, throughout the specification, the meaning of "a", "an", and "the" include plural references. The meaning of "in" includes "in" and "on."

As used herein, a "nucleic acid" or "nucleic acid molecule" generally refers to any ribonucleic acid or deoxyribonucleic acid, which may be unmodified or modified DNA or RNA. "Nucleic acids" include, without limitation, single- and double-stranded nucleic acids. As used herein, the term "nucleic acid" also includes DNA as described above that contains one or more modified bases. Thus, DNA with a backbone modified for stability or for other reasons is a "nucleic acid". The term "nucleic acid" as it is used herein embraces such chemically, enzymatically, or metabolically modified forms of nucleic acids, as well as the chemical forms of DNA characteristic of viruses and cells, including for example, simple and complex cells.

The terms "oligonucleotide" or "polynucleotide" or "nucleotide" or "nucleic acid" refer to a molecule having two or more deoxyribonucleotides or ribonucleotides, preferably more than three, and usually more than ten. The exact size will depend on many factors, which in turn depends on the ultimate function or use of the oligonucleotide. The oligonucleotide may be generated in any manner, including chemical synthesis, DNA replication, reverse transcription, or a combination thereof. Typical deoxyribonucleotides for DNA are thymine, adenine, cytosine, and guanine. Typical ribonucleotides for RNA are uracil, adenine, cytosine, and guanine.

As used herein, the terms "locus" or "region" of a nucleic acid refer to a subregion of a nucleic acid, e.g., a gene on a chromosome, a single nucleotide, a CpG island, etc.

The terms "complementary" and "complementarity" refer to nucleotides (e.g., 1 nucleotide) or polynucleotides (e.g., a sequence of nucleotides) related by the base-pairing rules. For example, the sequence 5'-A-G-T-3' is complementary to the sequence 3'-T-C-A-5'. Complementarity may be "partial," in which only some of the nucleic acids' bases are matched according to the base pairing rules. Or, there may be "complete" or "total" complementarity between the nucleic acids. The degree of complementarity between nucleic acid strands effects the efficiency and strength of hybridization between nucleic acid strands. This is of particular importance in amplification reactions and in detection methods that depend upon binding between nucleic acids.

The term "gene" refers to a nucleic acid (e.g., DNA or RNA) sequence that comprises coding sequences necessary for the production of an RNA, or of a polypeptide or its precursor. A functional polypeptide can be encoded by a full length coding sequence or by any portion of the coding sequence as long as the desired activity or functional properties (e.g., enzymatic activity, ligand binding, signal transduction, etc.) of the polypeptide are retained. The term "portion" when used in reference to a gene refers to fragments of that gene. The fragments may range in size from a few nucleotides to the entire gene sequence minus one nucleotide. Thus, "a nucleotide comprising at least a portion of a gene" may comprise fragments of the gene or the entire gene.

The term "gene" also encompasses the coding regions of a structural gene and includes sequences located adjacent to the coding region on both the 5' and 3' ends, e.g., for a distance of about 1 kb on either end, such that the gene corresponds to the length of the full-length mRNA (e.g., comprising coding, regulatory, structural and other sequences). The sequences that are located 5' of the coding region and that are present on the mRNA are referred to as 5' non-translated or untranslated sequences. The sequences that are located 3' or downstream of the coding region and that are present on the mRNA are referred to as 3' non-translated or 3' untranslated sequences. The term "gene" encompasses both cDNA and genomic forms of a gene. In some organisms (e.g., eukaryotes), a genomic form or clone of a gene contains the coding region interrupted with non-coding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

In addition to containing introns, genomic forms of a gene may also include sequences located on both the 5' and 3' ends of the sequences that are present on the RNA transcript. These sequences are referred to as "flanking" sequences or regions (these flanking sequences are located 5' or 3' to the non-translated sequences present on the mRNA transcript). The 5' flanking region may contain regulatory sequences such as promoters and enhancers that control or influence the transcription of the gene. The 3' flanking region may contain sequences that direct the termination of transcription, posttranscriptional cleavage, and polyadenylation.

The term "wild-type" when made in reference to a gene refers to a gene that has the characteristics of a gene isolated from a naturally occurring source. The term "wild-type" when made in reference to a gene product refers to a gene product that has the characteristics of a gene product isolated from a naturally occurring source. The term "naturally-occurring" as applied to an object refers to the fact that an object can be found in nature. For example, a polypeptide or polynucleotide sequence that is present in an organism (including viruses) that can be isolated from a source in nature and which has not been intentionally modified by the hand of a person in the laboratory is naturally-occurring. A wild-type gene is often that gene or allele that is most frequently observed in a population and is thus arbitrarily designated the "normal" or "wild-type" form of the gene. In contrast, the term "modified" or "mutant" when made in reference to a gene or to a gene product refers, respectively, to a gene or to a gene product that displays modifications in sequence and/or functional properties (e.g., altered characteristics) when compared to the wild-type gene or gene product. It is noted that naturally-occurring mutants can be isolated; these are identified by the fact that they have altered characteristics when compared to the wild-type gene or gene product.

"Amplification" is a special case of nucleic acid replication involving template specificity. It is to be contrasted with non-specific template replication (e.g., replication that is templatedependent but not dependent on a specific template). Template specificity is here distinguished from fidelity of replication (e.g., synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

Amplification of nucleic acids generally refers to the production of multiple copies of a polynucleotide, or a portion of the polynucleotide, typically starting from a small amount of the polynucleotide (e.g., a single polynucleotide molecule, 10 to 100 copies of a polynucleotide molecule, which may or may not be exactly the same), where the amplification products or amplicons are generally detectable. Amplification of polynucleotides encompasses a variety of chemical and enzymatic processes. The generation of multiple DNA copies from one or a few copies of a target or template DNA molecule during a polymerase chain reaction (PCR) or a ligase chain reaction (LCR; see, e.g., U.S. Patent No. 5,494,810) are forms of amplification. Additional types of amplification include, but are not limited to, allele-specific PCR (see, e.g., U.S. Patent No. 5,639,611), assembly PCR (see, e.g., U.S. Patent No. 5,965,408), helicase-dependent amplification (see, e.g., U.S. Patent No. 7,662,594), Hot-start PCR (see, e.g., U.S. Patent Nos. 5,773,258 and 5,338,671), intersequence-specfic PCR, inverse PCR (see, e.g., Triglia, et alet al. (1988) Nucleic Acids Res., 16:8186), ligation-mediated PCR (see, e.g., Guilfoyle, R. et alet al., Nucleic Acids Research, 25:1854-1858 (1997); U.S. Patent No. 5,508,169), methylation-specific PCR (see, e.g., Herman, et al., (1996) PNAS 93(13) 9821-9826), miniprimer PCR, multiplex ligation-dependent probe amplification (see, e.g., Schouten, et al., (2002) Nucleic Acids Research 30(12): e57), multiplex PCR (see, e.g., Chamberlain, et al., (1988) Nucleic Acids Research 16(23) 11141-11156; Ballabio, et al., (1990) Human Genetics 84(6) 571-573; Hayden, et al., (2008) BMC Genetics 9:80), nested PCR, overlap-extension PCR (see, e.g., Higuchi, et al., (1988) Nucleic Acids Research 16(15) 7351-7367), real time PCR (see, e.g., Higuchi, et alet al., (1992) Biotechnology 10:413-417; Higuchi, et al., (1993) Biotechnology 11:1026-1030), reverse transcription PCR (see, e.g., Bustin, S.A. (2000) J. Molecular Endocrinology 25:169-193), solid phase PCR, thermal asymmetric interlaced PCR, and Touchdown PCR (see, e.g., Don, et al., Nucleic Acids Research (1991) 19(14) 4008; Roux, K. (1994) Biotechniques 16(5) 812-814; Hecker, et al., (1996) Biotechniques 20(3) 478-485). Polynucleotide amplification also can be accomplished using digital PCR (see, e.g., Kalinina, et al., Nucleic Acids Research. 25; 1999-2004, (1997); Vogelstein and Kinzler, Proc Natl Acad Sci USA. 96; 9236-41, (1999); International Patent Publication No. WO05023091A2; US Patent Application Publication No. 20070202525).

As used herein, the term "nucleic acid detection assay" refers to any method of determining the nucleotide composition of a nucleic acid of interest. Nucleic acid detection assay include but are not limited to, DNA sequencing methods, probe hybridization methods, enzyme mismatch cleavage methods (e.g., Variagenics, U.S. Pat. Nos. 6,110,684, 5,958,692, 5,851,770); polymerase chain reaction; branched hybridization methods (e.g., Chiron, U.S. Pat. Nos. 5,849,481, 5,710,264, 5,124,246, and 5,624,802); rolling circle replication (e.g., U.S. Pat. Nos. 6,210,884, 6,183,960 and 6,235,502); NASBA (e.g., U.S. Pat. No. 5,409,818); molecular beacon technology (e.g., U.S. Pat. No. 6,150,097); E-sensor technology (Motorola, U.S. Pat. Nos. 6,248,229, 6,221,583, 6,013,170, and 6,063,573); cycling probe technology (e.g., U.S. Pat. Nos. 5,403,711, 5,011,769, and 5,660,988); Dade Behring signal amplification methods (e.g., U.S. Pat. Nos. 6,121,001, 6,110,677, 5,914,230, 5,882,867, and 5,792,614); ligase chain reaction (e.g., Barnay Proc. Natl. Acad. Sci USA 88, 189-93 (1991)); and sandwich hybridization methods (e.g., U.S. Pat. No. 5,288,609).

The term "amplifiable nucleic acid" refers to a nucleic acid that may be amplified by any amplification method. It is contemplated that "amplifiable nucleic acid" will usually comprise "sample template."

The term "primer" refers to an oligonucleotide, whether occurring naturally as in a purified restriction digest or produced synthetically, that is capable of acting as a point of initiation of synthesis when placed under conditions in which synthesis of a primer extension product that is complementary to a nucleic acid strand is induced, (e.g., in the presence of nucleotides and an inducing agent such as a DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer, and the use of the method.

The term "probe" refers to an oligonucleotide (e.g., a sequence of nucleotides), whether occurring naturally as in a purified restriction digest or produced synthetically, recombinantly, or by PCR amplification, that is capable of hybridizing to another oligonucleotide of interest. A probe may be single-stranded or double-stranded. Probes are useful in the detection, identification, and isolation of particular gene sequences (e.g., a "capture probe"). It is contemplated that any probe used in the present invention may, in some embodiments, be labeled with any "reporter molecule," so that is detectable in any detection system, including, but not limited to enzyme (e.g., ELISA, as well as enzyme-based histochemical assays), fluorescent, radioactive, and luminescent systems. It is not intended that the present invention be limited to any particular detection system or label.

As used herein, the term "neoplasm" refers to "an abnormal mass of tissue, the growth of which exceeds and is uncoordinated with that of the normal tissues" See, e.g., Willis RA, "The Spread of Tumors in the Human Body", London, Butterworth & Co, 1952.

A "site" of a neoplasm, adenoma, cancer, etc. is the tissue, organ, cell type, anatomical area, body part, etc. in a subject's body where the neoplasm, adenoma, cancer, etc. is located.

As used herein, a "diagnostic" test application includes the detection or identification of a disease state or condition of a subject, determining the likelihood that a subject will contract a given disease or condition, determining the likelihood that a subject with a disease or condition will respond to therapy, determining the prognosis of a subject with a disease or condition (or its likely progression or regression), and determining the effect of a treatment on a subject with a disease or condition. For example, a diagnostic can be used for detecting the presence or likelihood of a subject contracting a neoplasm or the likelihood that such a subject will respond favorably to a compound (e.g., a pharmaceutical, e.g., a drug) or other treatment.

The term "isolated" when used in relation to a nucleic acid, as in "an isolated oligonucleotide" refers to a nucleic acid sequence that is identified and separated from at least one contaminant nucleic acid with which it is ordinarily associated in its natural source. Isolated nucleic acid is present in a form or setting that is different from that in which it is found in nature. In contrast, non-isolated nucleic acids, such as DNA and RNA, are found in the state they exist in nature. Examples of non-isolated nucleic acids include: a given DNA sequence (e.g., a gene) found on the host cell chromosome in proximity to neighboring genes; RNA sequences, such as a specific mRNA sequence encoding a specific protein, found in the cell as a mixture with numerous other mRNAs which encode a multitude of proteins. However, isolated nucleic acid encoding a particular protein includes, by way of example, such nucleic acid in cells ordinarily expressing the protein, where the nucleic acid is in a chromosomal location different from that of natural cells, or is otherwise flanked by a different nucleic acid sequence than that found in nature. The isolated nucleic acid or oligonucleotide may be present in single-stranded or double-stranded form. When an isolated nucleic acid or oligonucleotide is to be utilized to express a protein, the oligonucleotide will contain at a minimum the sense or coding strand (i.e., the oligonucleotide may be single-stranded), but may contain both the sense and anti-sense strands (i.e., the oligonucleotide may be double-stranded). An isolated nucleic acid may, after isolation from its natural or typical environment, by be combined with other nucleic acids or molecules. For example, an isolated nucleic acid may be present in a host cell in which into which it has been placed, e.g., for heterologous expression.

The term "purified" refers to molecules, either nucleic acid or amino acid sequences that are removed from their natural environment, isolated, or separated. An "isolated nucleic acid sequence" may therefore be a purified nucleic acid sequence. "Substantially purified" molecules are at least 60% free, preferably at least 75% free, and more preferably at least 90% free from other components with which they are naturally associated. As used herein, the terms "purified" or "to purify" also refer to the removal of contaminants from a sample. The removal of contaminating proteins results in an increase in the percent of polypeptide or nucleic acid of interest in the sample. In another example, recombinant polypeptides are expressed in plant, bacterial, yeast, or mammalian host cells and the polypeptides are purified by the removal of host cell proteins; the percent of recombinant polypeptides is thereby increased in the sample.

The term "sample" is used in its broadest sense. In one sense it can refer to an animal cell or tissue. In another sense, it is meant to include a specimen or culture obtained from any source, as well as biological and environmental samples. Biological samples may be obtained from plants or animals (including humans) and encompass fluids, solids, tissues, and gases. Environmental samples include environmental material such as surface matter, soil, water, and industrial samples. These examples are not to be construed as limiting the sample types applicable to the present invention.

As used herein, the terms "patient" or "subject" refer to organisms to be subject to various tests provided by the technology. The term "subject" includes animals, preferably mammals, including humans. In a preferred embodiment, the subject is a primate. In an even more preferred embodiment, the subject is a human.

### Examples of the technology

The following is presented by way of example and not by way of limitation.

Currently, breast conservation therapy for patients with localized invasive breast cancer includes surgical resection followed by radiation therapy, with chemotherapy given to selected patients. While molecular prognostic tools, such as OncotypeDx, can be used to help guide decisions regarding chemotherapy in subsets of breast cancer patients, no similar tools exist to inform decisions regarding radiation therapy. Therefore, there is a clear need to identify the inherent radiation sensitivity of each patient's breast cancer, and to develop approaches of combining targeted drugs with radiation therapy for patients with aggressive tumors that have a high chance of local recurrence following radiation alone.

To this end, provided herein are radiation sensitivity signatures, referred to herein as RadiotypeDx. These signatures are based on the expression of genes that distinguish between radiation sensitive (also referred to as radiosensitive) and radiation-resistant (also referred to as radioresistant) cancer cells and subject (e.g., breast cancer). The following description is focused on breast cancer for illustrative purposes. However, it should be understood that the technology may be applied to a variety of cancer types.

Breast cancer is the most common form of cancer in women and the second most common cause of cancer-related death. Traditionally breast cancer has been treated with surgery, radiation, and chemotherapy. While these modalities are effective for many early stage breast cancers, especially estrogen receptor (ER)-positive breast cancer, many breast cancers (including a disproportionately large number of ER-negative breast cancers) still recur. Multiple randomized clinical trials have demonstrated the benefit of adjuvant radiation therapy after breast conserving surgery. However, it is clear that current adjuvant radiotherapy approaches result in overtreatment of up to 70% of patients who are unlikely to recur after surgery alone. Conversely, there are subsets of patients (up to 20%) who, despite standard multimodality treatment including radiation, will develop local recurrence. Thus, there is a clear need to identify these two populations: those who are currently overtreated, and those who need further treatment intensification. Additionally, for those that are likely to develop recurrence despite standard treatment, further efforts should be aimed at developing molecularly targeted therapies that are more effective and less toxic than currently available treatment option.

Recent gene expression profiling studies in breast cancer have identified clinically significant heterogeneity amongst breast tumors in terms of gene and protein expression that is not fully accounted for by the standard histopathologic classification of breast cancer. Furthermore, studies detailing the poor response of basal-like (often ER-negative, PR-negative, and HER2/*neu* negative) and HER2/*neu* positive tumors to adjuvant radiation therapy further underscore the biologic differences and as yet undefined oncogenic drivers of these particular types of tumors, with basal-like and HER2/*neu* positive tumors much less likely to have significant disease free and overall survival advantages from adjuvant radiation treatment in women at high risk for local recurrence. Therefore, there is a clear need to identify the radiation sensitivity of each patient's breast cancer, and to develop approaches of combining targeted drugs with radiation therapy for patients with aggressive tumors that have a high chance of local recurrence following radiation alone.

Unfortunately there has been a relative absence to clinically available radiation sensitizers in women with treatment refractory breast cancers or for women who are at high risk of local recurrence given their clinicopathologic characteristics. Recent collaborative efforts have begun to identify not only recurrent mutations in breast cancer, but have also begun to assess intrinsic sensitivity to chemotherapeutic and molecularly targeted treatments based on these mutations. This information, made publically available, provides a valuable resource for pathway and target discovery within the context of traditionally treatment-refractory disease. Given the lack of targeted agents for triple-negative disease and their relative radiation insensitivity, as evidenced by their increased locoregional recurrence risk, it is clear that additional targets for radiosensitization are critically needed.

Currently it is not possible to identify *a priori* which patients are likely to develop a recurrence after adjuvant radiation therapy (i.e. those with radioresistant disease) for whom treatment intensification may be indicated. Despite this clinical limitation, there is precedent for the development of such a test to predict patients likely to benefit from adjuvant treatment. Indeed, molecular prognostic tools, such as OncotypeDx, can be used to help guide decisions regarding chemotherapy in subsets of breast cancer patients. This gene expression based test relies on the expression values of 16 cancer related genes to not only be prognostic in women with estrogen receptor-positive, node positive disease, but is also predictive of response to adjuvant chemotherapy. While this test is currently utilized in clinical practice to guide treatment decisions regarding adjuvant chemotherapy, no such similar tools exist to inform decisions regarding radiation therapy.

To this end, provided herein is a radiation sensitivity signature, that can distinguish between radiation-sensitive and radiation-resistant subjects. This finds use in identifying subject suitable and not suitable for radiotherapy and also finds use to identify and select therapeutic strategies that overcome radiation resistance. As described in the Examples section below, radiation sensitivity and resistance signature were generated that find use to predict the necessity and utility of postsurgical adjuvant radiation treatment. This has been validated in several nonrandomized experiments. Based on such signatures, diagnostic tests (e.g., PCR platform, nanostring platform, or other desired platform etc.) are used to calculates a recurrence score that finds use to guide clinical decision making in deciding which patients benefit from adjuvant radiation therapy.

Experiments conducted during development of embodiments described herein demonstrate significant heterogeneity in the intrinsic breast cancer radiosensitivity of breast cancers and demonstrate that this radiosensitivity is independent of intrinsic breast cancer subtype. Based on this heterogeneity, a molecular signature of radiation response in breast cancer was developed that is enriched for biologic concepts implicated in response to radiation therapy including DNA damage repair and cell cycle regulation. Furthermore, genes previously unreported to be associated with radiation sensitivity were identified, and it was demonstrated that perturbation of these genes is sufficient to confer alterations in the response to radiation. Experimental evidence using an independent dataset demonstrates the prognostic import of this signature. Radiation response signatures described herein are able to, with great sensitivity and specificity, discriminate patients unlikely to develop local recurrence after radiation therapy from those patients at high likelihood of recurrence despite standard radiation therapy. Signatures described herein may be local recurrence molecular signatures, and may be also prognostic for overall survival, consistent with the finding in breast cancer-specific metaanalyses that local recurrence benefit translates into an overall survival advantage in human patients (Early Breast Cancer Trialists' Collaborative.. Lancet, 2011. 378(9804): p. 1707-16; Clarke, M., et al., Lancet, 2005. 366(9503): p. 2087-106).

Provided herein are breast cancer-specific molecular signatures of radiation response that provides potentially clinically relevant data regarding the prognosis of patients who receive adjuvant radiation therapy for management of breast cancer. Despite previous attempts to generate a radiation response signature, no single signature has performed well in validation using cohorts of patients with breast cancer. The failure of previous attempts is not surprising, given the diversity and heterogeneity of genomic, transcriptomic, and proteomic alterations common to cancers of different origins. Recent analysis of the mutation landscapes across various cancer types further characterizes the heterogeneity of various cancer types (Kandoth, C., et al., Nature, 2013. 502(7471): p. 333-9.; Lawrence, M.S., et al., Nature, 2014. 505(7484): p. 495-501.). Thus, previous signature attempts which rely on radiation sensitivity data from a diverse range of human cancers may have identified conserved pathways implicated in generic cellular response to ionizing radiation, but these have performed poorly in individual cancer types, including breast cancer. Previous studies demonstrated that the clinical and biological features of human breast tumors and human breast cancer cell lines derived from these tumors is remarkably well conserved (Neve, R.M., et al., Cancer Cell, 2006. 10(6): p. 515-27.; Wistuba, II, et al., Clin Cancer Res, 1998. 4(12): p. 2931-8.). Capitalizing on these similarities experiments were conducted utilizing the intrinsic radiosensitivity of human breast cancer cell lines to develop a radiation response signature that is breast cancer specific. The radiation sensitivity of human breast cancer cell lines is independent of intrinsic breast cancer subtype and therefore is not a recapitulation of the previously described subtypes.

Breast cancer-specific molecular signatures of radiation response, and methods of use thereof find use with a variety of subjects. Such subject include, but are not limited to, all subjects diagnosed with DCIS or LCIS, any subjects treated with breast conserving therapy, including those with 0-3 LN positive; subjects with advanced disease treated with mastectomy with positive LN or 1-3 LN positive.

The technology relates to assessing the expression of combinations of markers comprising, e.g., 2, 3, 4, 5, 6, 7, 8, 9, 10, 11, 12, 13, 14, 15, 16, 17, 18, 19, 20, 21, 22, 23, 24, 25, 26, 27, 27, 29, 30, or more markers, and ranges therein (e.g., from Table 1, Table 2, Table 5, and/or Table 6). Levels of two or more markers from one or more of Tables 1, 2, 5, and 6 may be analyzed in combination with one or more other markers (e.g., breast cancer markers, radiosensitiveity/radioresistance markers, cancer markers, chemosensitivity/chemoresistance markers, unrelated markers). Levels of two or more markers from one or more of Tables 1, 2, 5, and 6 may be analyzed with fewer than 500 total markers (e.g., <500, <400, <300, <200, <100, <50, <40, <30, <20, <10). A panel or multiplex assay or multiple assays may be conducted that assess the expression of two or more markers from Table 1, two or more markers from Table 2, two or more markers from Table 5, and/or two or more markers from Table 6. The expression results are analyzed to generate a risk score (e.g., via computer algorithm weighing each of the markers' expression levels and, for example, comparing to a look-up table of established risk associated with such marker or expression level; in some embodiments, sub-categorized by patient sub-type (e.g., based on age, gender, disease type, or other desired factor)). Markers may be differentially weighted in order to accurately generate a risk score.

Assessing the expression of more than one marker may increase the specificity and/or sensitivity of a screen or diagnostic. A marker or a combination of markers may discriminate between subjects likely responsive or unresponsive to a particular therapy (e.g., radiotherapy). Patient responses are predicted by various combinations of markers, e.g., as identified by statistical techniques. The technology provides methods for identifying predictive combinations and validated predictive combinations of markers. Markers described in Table 1, Table 2, Table 5, and/or Table 6 may be employed.

Nucleic acid expression may be assessed by any desired technique. Nucleic acid (e.g., RNA) may be first isolated from a sample. Nucleic acid may be isolated by any means, including the use of commercially available kits. Briefly, wherein the nucleic acid of interest is encapsulated in by a cellular membrane the biological sample may be disrupted and lysed by enzymatic, chemical or mechanical means. The nucleic acid is then recovered from the solution. This may be carried out by means of a variety of methods including salting out, organic extraction, or binding of the nucleic acid to a solid phase support. The choice of method will be affected by several factors including time, expense, and required quantity and/or quality of nucleic acid desired. All clinical sample types are suitable for use in the present method, e.g., cell lines, histological slides, biopsies, paraffin-embedded tissue, body fluids, stool, colonic effluent, urine, blood plasma, blood serum, whole blood, isolated blood cells, cells isolated from the blood, and combinations thereof.

The technology relates to a method for treating a patient (e.g., a patient with cancer), the method comprising determining the expression level of one or more markers as provided herein and administering a treatment to the patient based on the results of analysis. However said treatment in not part of the claimed invention in view of Art. 53(c) EPC. The treatment may be administration of radiotherapy (alone or in combination with other therapies) or selection of a non-radiotherapy, including, but not limited to, use of a pharmaceutical compound, a vaccine, performing a surgery, etc. The markers may also be used to monitor a patient during a course of therapy to determine, for example, whether the therapy is or remains or become efficacious and to determine whether changes in therapy should be made.

Contemplated herein are any methods capable of receiving, processing, and transmitting the information to and from laboratories conducting the assays of radiosensitivity, information providers, medical personal, and subjects (e.g., from which cell sample are taken). For example, a sample (e.g., a biopsy or a blood or serum sample) is obtained from a subject (e.g., by a clinician) and submitted to a profiling service (e.g., clinical lab at a medical facility, genomic profiling business, third party testing service/facility, etc.), located in any part of the world to generate raw data. The subject may visit a medical center to have the sample obtained and sent to the profiling center. Once received by the profiling service, the sample is processed and a profile is produced (e.g., expression data), specific for the diagnostic or prognostic information desired for the subj ect.

The profile data is then prepared in a suitable format (e.g., suitable for interpretation by a treating clinician). For example, rather than providing raw expression data (e.g., expression levels of a subset of genes from Tables 1, 2, 5, and/or 6), the prepared format may represent a diagnosis or risk assessment, along with recommendations for particular treatment options. The data may be displayed to the clinician by any suitable method. For example, the profiling service generates a report that can be printed for the clinician (e.g., at the point of care) or displayed to the clinician on a computer monitor.

The information may be first analyzed at the point of care or at a regional facility. The raw data is then sent to a central processing facility for further analysis and/or to convert the raw data to information useful for a clinician or patient. The central processing facility provides the advantage of privacy (all data is stored in a central facility with uniform security protocols), speed, and uniformity of data analysis. The central processing facility can then control the fate of the data following treatment of the subject. For example, using an electronic communication system, the central facility can provide data to the clinician, the subject, or researchers.

All or a portion of the methods described herein may beprovided as a service. A user (e.g., subject (e.g., patient), clinician, researcher, etc.) may arrange, contract, pay, etc. to have a sample (e.g., comprising a population of cells) and/or data (e.g., raw data) analyzed. A sample may be submitted (e.g., in-person, via mail or courier, etc.) and analysis of the sample for specific biomarkers described herein (e.g., a subset of genes from Tables 1, 2, 5, and/or 6), alone or with other biomarkers, may be performed by the service (e.g., at a diagnostic testing facility, etc.). Data collected by a user (e.g., a clinician, researcher, etc.) may be submitted to a testing facility for analysis. Examples described herein include any suitable combination of user-performed (e.g., subject-performed, clinician-performed, etc.) and service-performed steps. Methods described herein may comprise or may consist of only the steps performed by either the user (e.g., subject, clinician, etc.) of the service (e.g., sample collection, sending a sample, sample analysis, data collection, data analysis, receiving a report, etc.), or the service (e.g., sample collection, receiving a sample, sample analysis, data analysis, generating a report, sending a report, etc.). Any combination of steps may be performed by a user and/or service.

Analysis results may be reported (e.g., to a health care professional, to a subject, etc.). A result may be provided on a peripheral, device, or component of an apparatus. For example, sometimes an outcome is provided by a printer or display. An outcome may be reported in the form of a report, and in certain examples the report comprises biomarker levels, risk assessment, a compiled score (e.g., based on a plurality of markers), etc. An outcome can be translated into and displayed in a suitable format that facilitates downstream use of the reported information.

Generating and reporting results from the raw biomarker data (e.g., expression levels) may comprise transformation of the data reads into a representation that reflects information not determinable in the absence of the method steps described herein. Converting biomarker levels into useful information allows actions to be taken (e.g., in response to identifying the radiosensitivity of a cell population (e.g., from a subject)). As such, the methods provided herein address the problem of identifying the radiosensitivity of populations of cells (e.g., cancer cells) from subjects that confronts the fields of medicine, public health, public policy, etc.

A user or a downstream individual, upon receiving or reviewing a report comprising one or more results determined from the analyses provided herein, may take specific steps or actions in response. For example, a health care professional or qualified individual may provide further testing of a subject (e.g., the subject from which a biological sample comprising the tested cells was obtained). There is no limitation by the number of ways or fields in which the technology herein may find use.

The term "receiving a report" as used herein refers to obtaining, by a communication means, a written and/or graphical representation comprising results or outcomes of the biomarker analysis described herein. The report may be generated by a computer or by human data entry, and can be communicated using electronic means (e.g., over the internet, via computer, via fax, from one network location to another location at the same or different physical sites), or by another method of sending or receiving data (e.g., mail service, courier service and the like). The outcome may be transmitted in a suitable medium, including, without limitation, in verbal, document, or file form. The file may be, for example, but not limited to, an auditory file, a computer readable file, a paper file, a laboratory file or a medical record file. A report may be encrypted to prevent unauthorized viewing.

As noted above, systems and method described herein may transform data from one form into another form (e.g., from physical biomarkers in a sample to actual diagnosis, etc.). The terms "transformed", "transformation", and grammatical derivations or equivalents thereof, refer to an alteration of data from a physical starting material (e.g., nucleic acid or protein in a biological sample, etc.) into a digital representation of the physical starting material (e.g., read data), a representation of the amount of that starting material (e.g., biomarker level), a condensation of the sequential representation (e.g., a combined signature based on multiple biomarkers), or a diagnosis, prognosis, or risk assessment, etc. Transformation may involve conversion of data between any of the above.

### EXPERIMENTAL

### Example 1

Experiments were conducted during development of embodiments of the present invention to identify a radiosensitivity gene signature, using publicly available gene expression datasets from multiple cohorts of breast cancer samples from patients treated with radiotherapy with long-term clinical follow-up.

Using clonogenic survival assays, the range of surviving fraction (SF) after 2 Gy of radiotherapy (RT) across 21 BC cell lines was determined (SEE FIG. 1). Using SF as a continuous variable, the RT sensitivity score (RSS) was correlated to gene expression using a Spearman correlation method on an individual gene basis. Genes were selected for the signature based on positive or negative correlation. Unsupervised hierarchical clustering identified (SEE FIG. 2) differences in gene expression across resistant and sensitive cell lines to generate a radiation sensitivity (RS) signature. This signature was trained and validated in a separate human breast tumor dataset containing early stage, node-negative patients treated with surgery and RT alone without adjuvant chemotherapy to assess the predictive effect of RS signature on recurrence risk after RT. Gene function and potentially actionable targets from the signature were validated using clonogenic survival and DNA damage assays.

Clonogenic survival identifies a range of radiation sensitivity in human BCC lines (SF 77%-17%) with no significant correlation to the intrinsic BC subtype. Using a 2-sided Spearmans correlation method, a total of 126 genes were identified as being associated with radiation sensitivity (positively correlated (Table 1), negatively correlated (Table 2)). Unsupervised hierarchical expression discriminates gene expression patterns in the RT resistant and RT sensitive cell lines and is enriched for genes involved in cell cycle arrest and DNA damage response. Knockdown of genes associated with the radioresistance signature identifies previously unreported radiation resistance genes, including TACC1 and RND3 with enhancement ratios of 1.25 and 1.37 in BCC lines. Application of this RS signature to an independent breast cancer dataset with clinical outcomes validates the signature and accurately identifies patients with decreased rates of recurrence compared to patients with high expression of the radioresistant signature. All or a portion of the negatively and positively correlated genes may serve as a predictive signature of research and clinical utility.

**Table 1 - Radiosensitivity Associate Genes**

| **Gene Name** | **Gene ID** |
|---|---|
| Abhydrolase domain containing 11 | ABHD11 |
| Anterior gradient homolog 2 (Xenopus laevis) | AGR2 |
| Apolipoprotein D | APOD |
| | ARHGAP8 |
| ASF1 anti-silencing function 1 homolog B (S. cerevisiae) | ASF1B |
| B-cell CLL/lymphoma 7C | BCL7C |
| BEX family member 4 | BEX4 |
| Bloom syndrome | BLM |
| BTG family, member 2 | BTG2 |
| BUB 1 budding uninhibited by benzimidazoles 1 homolog | BUB1B |
| Chromosome 19 open reading frame 21 | C19orf21 |
| Chromosome 22 open reading frame 9 | C22orf9 |
| Chromosome 4 open reading frame 19 | C4orf19 |
| | CALHM2 |
| | CDC45 |
| Cyclin-dependent kinase inhibitor 1A (p21, Cip1) | CDKN1A |
| Chromatin licensing and DNA replication factor 1 | CDT1 |
| Cordon-bleu homolog (mouse) | COBL |
| Desmoplakin | DSP |
| Denticleless homolog (Drosophila) | DTL |
| Eukaryotic translation initiation factor 1A, X-linked | EIF1AX |
| Epithelial membrane protein 2 | EMP2 |
| Extra spindle pole bodies homolog 1 (S. cerevisiae) | ESPL1 |
| Fatty acid 2-hydroxylase | FA2H |
| FERM, RhoGEF (ARHGEF) and pleckstrin domain protein 1 | FARP1 |
| Fructose-1,6-bisphosphatase 1 | FBP1 |
| Glucosaminyl (N-acetyl) transferase 1, core 2 | GCNT1 |
| | GIYD1 |
| | GLB1L2 |
| Glutathione S-transferase M3 (brain) | GSTM3 |
| Histamine N-methyltransferase | HNMT |
| Hook homolog 1 (Drosophila) | HOOK1 |
| Homeobox C13 | HOXC13 |
| Interferon stimulated exonuclease gene 20kDa | ISG20 |
| Kinesin family member 11 | KIF11 |
| Kinesin family member 14 | KIF 14 |
| Ladinin 1 | LAD1 |
| LIM and cysteine-rich domains 1 | LMCD1 |
| MARCKS-like 1 | MARCKSL1 |
| Minichromosome maintenance complex component 10 | MCM10 |
| Minichromosome maintenance complex component 2 | MCM2 |
| Minichromosome maintenance complex component 5 | MCM5 |
| Microfibrillar-associated protein 3-like | MFAP3L |
| Monoglyceride lipase | MGLL |
| O-6-methylguanine-DNA methyltransferase | MGMT |
| Antigen identified by monoclonal antibody Ki-67 | MKI67 |
| MKL/myocardin-like 2 | MKL2 |
| Msh homeobox 1 | MSX1 |
| Metastasis suppressor 1 | MTSS1 |
| Myosin VC | MYO5C |
| Neurogranin (protein kinase C substrate, RC3) | NRGN |
| Neurturin | NRTN |
| Progestin and adipoQ receptor family member IV | PAQR4 |
| Programmed cell death 4 (neoplastic transformation inhibitor) | PDCD4 |
| PDZ domain containing 2 | PDZD2 |
| Phosphoinositide-3-kinase, regulatory subunit 3 (p55, gamma) | PIK3R3 |
| Protein kinase, membrane associated tyrosine/threonine 1 | PKMYT1 |
| Plakophilin 1 (ectodermal dysplasia/skin fragility syndrome) | PKP1 |
| Phosphatidylinositol-specific phospholipase C, X dom. 1 | PLCXD1 |
| Pleckstrin homology domain FM | PLEKHA1 |
| Podocalyxin-like | PODXL |
| Polymerase (DNA directed), epsilon 2 (p59 subunit) | POLE2 |
| Polymerase (RNA) III (DNA directed) polypeptide K, 12.3 kDa | POLR3K |
| Protein regulator of cytokinesis 1 | PRC1 |
| Protein kinase, X-linked | PRKX |
| Proline dehydrogenase (oxidase) 1 | PRODH |
| Phosphoribosyl pyrophosphate synthetase 2 | PRPS2 |
| RAB15, member RAS onocogene family | RAB15 |
| RAB GTPase activating protein 1-like | RABGAP1L |
| RAD51 associated protein 1 | RAD51AP1 |
| SIVA1, apoptosis-inducing factor | SIVA1 |
| S-phase kinase-associated protein 2 (p45) | SKP2 |
| Solute carrier family 16, member 7 | SLC16A7 |
| | SNRNP25 |
| SPC25, NDC80 kinetochore complex component, homolog | SPC25 |
| Small proline-rich protein 1B (cornifin) | SPRR1B |
| ST3 beta-galactoside alpha-2,3-sialyltransferase 5 | ST3GAL5 |
| Serine/threonine kinase 38 like | STK38L |
| Transducin (beta)-like 1X-linked | TBL1X |
| Thymidine kinase 1, soluble | TK1 |
| Tetraspanin 13 | TSPAN13 |
| Tetraspanin 8 | TSPAN8 |
| Thioredoxin interacting protein | TXNIP |

**Table 2 - Radioresistance Associate Genes**

| **Gene Name** | **Gene ID** |
|---|---|
| adhesion molecule with Ig-like domain 2 | AMIGO2 |
| angiomotin like 2 | AMOTL2 |
| hypothetical LOC100129500; apolipoprotein E | APOE |
| armadillo repeat containing, X-linked 2 | ARMCX2 |
| butyrylcholinesterase | BCHE |
| chromosome 14 open reading frame 139 | C14orf139 |
| CCAAT/enhancer binding protein (C/EBP), beta | CEBPB |
| collagen, type XVI, alpha 1 | COL16A1 |
| cullin 4B | CUL4B |
| chemokine (C-X-C motif) ligand 6 (granulocyte chemotactic protein 2) | CXCL6 |
| cysteine-rich, angiogenic inducer, 61 | CYR61 |
| doublecortin-like kinase 1 | DCLK1 |
| discoidin domain receptor tyrosine kinase 2 | DDR2 |
| degenerative spermatocyte homolog 1, lipid desaturase (Drosophila) | DEGS1 |
| deiodinase, iodothyronine, type II | DIO2 |
| deleted in lymphocytic leukemia 1 (non-protein coding) | DLEU1 |
| distal-less homeobox 2 | DLX2 |
| formin homology 2 domain containing 3 | FHOD3 |
| formin binding protein 1 | FNBP1 |
| frizzled homolog 2 (Drosophila) | FZD2 |
| galactosylceramidase | GALC |
| glucan (1,4-alpha-), branching enzyme 1 | GBE1 |
| golgi autoantigen, golgin subfamily | GOLGA8A |
| golgi autoantigen, golgin subfamily a, 8B | GOLGA8B |
| glutathione peroxidase 7 | GPX7 |
| HEG homolog 1 (zebrafish) | HEG1 |
| hect domain and RLD 2 pseudogene 2 | HERC2P2 |
| hect domain and RLD 2 pseudogene 2 | HERC2P3 |
| HIV-1 Tat interactive protein 2, 30kDa | HTATIP2 |
| intercellular adhesion molecule 1 | ICAM1 |
| interferon gamma receptor 1 | IFNGR1 |
| inositol 1,4,5-triphosphate receptor, type 1 | ITPR1 |
| potassium channel, subfamily K, member 2 | KCNK2 |
| KIAA0802 | KIAA0802 |
| kin of IRRE like (Drosophila) | KIRREL |
| lysosomal-associated membrane protein 2 | LAMP2 |
| hect domain and RLD 2 pseudogene | LOC440248 |
| latent transforming growth factor beta binding protein 2 | LTBP2 |
| melanoma antigen family A, 3 | MAGEA3 |
| mediator complex subunit 17 | MED17 |
| matrix metallopeptidase 2 | MMP2 |
| M-phase phosphoprotein 8 | MPHOSPH8 |
| nuclear factor, interleukin 3 regulated | NFIL3 |
| oncostatin M receptor | OSMR |
| osteopetrosis associated transmembrane protein 1 | OSTM1 |
| pre-B-cell leukemia homeobox 3 | PBX3 |
| platelet derived growth factor C | PDGFC |
| popeye domain containing 3 | POPDC3 |
| protein kinase C, alpha | PRKCA |
| ProSAPiPl protein | ProSAPiP1 |
| phosphoserine phosphatase-like; phosphoserine phosphatase | PSPH |
| prostaglandin E receptor 4 (subtype EP4) | PTGER4 |
| peroxisomal membrane protein 4, 24kDa | PXMP4 |
| RAB31, member RAS oncogene family | RAB31 |
| REX2, RNA exonuclease 2 homolog (S. cerevisiae) | REXO2 |
| regulator of G-protein signaling 4 | RGS4 |
| Rho family GTPase 3 | RND3 |
| ring finger protein (C3H2C3 type) 6 | RNF6 |
| regulation of nuclear pre-mRNA domain containing 1A | RPRD1A |
| secreted and transmembrane 1 | SECTM1 |
| serpin peptidase inhibitor, clade E member 2 | SERPINE2 |
| solute carrier family 39 (zinc transporter), member 14 | SLC39A14 |
| SH3 and cysteine rich domain | STAC |
| serine/threonine kinase 17a | STK17A |
| transforming, acidic coiled-coil containing protein 1 | TACC1 |
| transcription factor 4 | TCF4 |
| transcription factor CP2 | TFCP2 |
| transmembrane protein 30A | TMEM30A |
| thioredoxin-related transmembrane protein 4 | TMX4 |
| TRAF2 and NCK interacting kinase | TNIK |
| tripeptidyl peptidase I | TPP1 |
| ubiquitin specific peptidase like 1 | USPL1 |
| vesicle-associated membrane protein 7 | VAMP7 |
| Yes-associated protein 1, 65kDa | YAP1 |
| zinc finger protein 259 | ZNF259 |
| zinc finger protein 259, pseudogene | ZNF259P1 |
| similar to zinc finger protein 347; zinc finger protein 532 | ZNF532 |

The expression of selected genes identified in array profiling were validated as being more highly expressed in the radiation resistant cell lines compared to the radiation sensitive cell lines by western blotting (SEE FIG. 3A). RNA Expression data for 2 representative genes (*TACC1 and RND3)* from a panel or radiation resistant and radiation sensitive breast cancer cell lines are shown were analyzed (SEE FIG. 3B).

Knockdown experiments with siRNA designed against TACC-1, a gene identified in the radioresistance signature, effectively knocked down the expression of TACC-1 (SEE FIG. 4A). Clonogenic survival assays in MDA-MB-231 breast cancer cells after siRNA knockdown of TACC-1 show significant sensitization of these cells to ionizing radiation with an enhancement ratio of 1.1-1.4.

Genes identified in experiments conducted during development of embodiments of the present invention were sorted by gene function to identify gene functions enriched in genes negatively associated with clonogenic survival (Table 3) and gene functions enriched in genes positively associated with clonogenic survival (Table 4).

**Table 3 - Gene Functions Enriched in Genes Negatively Associated with Clonogenic Survival**

| **Feature** | **FDR** | **Genes in network** | **Genes in genome** |
|---|---|---|---|
| M/G1 transition of mitotic cell cycle | 5.02E-14 | 13 | 79 |
| cell cycle checkpoint | 4.52E-12 | 16 | 235 |
| regulation of cell cycle arrest | 1.10E-11 | 16 | 255 |
| G1/S transition of mitotic cell cycle | 1.19E-11 | 14 | 169 |
| S phase | 4.64E-10 | 12 | 135 |
| DNA replication | 1.09E-09 | 13 | 191 |
| S phase of mitotic cell cycle | 5.44E-09 | 11 | 128 |
| DNA-dependent DNA replication | 1.90E-06 | 8 | 81 |
| DNA strand elongation involved in DNA replication | 2.82E-04 | 5 | 32 |
| DNA strand elongation | 4.06E-04 | 5 | 35 |
| DNA-dependent DNA replication initiation | 1.28E-03 | 4 | 19 |
| regulation oftranscription involved in G1/S phase of mitotic cell cycle | 1.46E-03 | 4 | 20 |
| DNA replication checkpoint | 9.36E-03 | 3 | 10 |
| negative regulation of G2/M transition of mitotic cell cycle | 1.48E-02 | 3 | 12 |
| DNA integrity checkpoint | 1.48E-02 | 6 | 132 |
| G2/M transition of mitotic cellcycle | 1.48E-02 | 6 | 134 |
| nuclear chromosome part | 5.91E-02 | 6 | 173 |
| ATP-dependent DNA helicase activity | 9.12E-02 | 3 | 23 |
| regulation of G2/M transition of mitotic cellcycle | 9.12E-02 | 3 | 23 |

**Table 4 - Gene Functions Enriched in Genes Positively Associated with Clonogenic Survival**

| **Feature** | **FDR** | **Genes in network** | **Genes in genome** |
|---|---|---|---|
| tRNA aminoacylation for protein translation | 2.71E-01 | 4 | 42 |
| amino acid activation | 2.71E-01 | 4 | 45 |
| transforming growth factor beta receptor signaling pathway | 2.71E-01 | 5 | 88 |
| tRNA aminoacylation | 2.71E-01 | 4 | 45 |
| regulation of platelet activation | 2.91E-01 | 3 | 18 |
| aminoacyl-tRNA ligase activity ligase activity, forming aminoacyl-tRNA and related compounds | 4.42E-01 | 3 | 24 |
| | 4.42E-01 | 3 | 24 |
| ligase activity, forming carbon-oxygen bonds | 4.42E-01 | 3 | 24 |
| positive regulation of fibroblast proliferation | 5.01E-01 | 3 | 26 |
| tRNA metabolic process | 5.46E-01 | 4 | 68 |

### Example 2

Using clonogenic survival assays, the range of surviving fraction after 2 Gy (SF-2Gy) of radiation was identified across 16 breast cancer cell lines. Using SF-2Gy as a continuous variable, the intrinsic radiation sensitivity was correlated to gene expression using Spearman's correlation method. Genes were selected for the signature based on significant positive or negative correlation of radiation naive expression with SF-2Gy. Using these genes, a radiation sensitivity signature was generated using a Random Forest model and was refined, cross-validated, and then independently validated in multiple separate human breast tumor datasets.

Clonogenic survival identifies a range of radiation sensitivity in human breast cancer cell lines (SF-2Gy 77%-17%) with no significant correlation (r value <0.3) to the intrinsic breast cancer subtype. Using Spearman's correlation method, a total of 147 genes were identified as being associated with radiation sensitivity (80 positively correlated, 67 negatively correlated (See Table 5)). Functional analysis of signature genes identifies previously unreported radiation resistance-associated genes, including TACC1 and RND3. The radiation sensitivity signature was trained and further refined to 51 genes (See Table 6) that were enriched for biological concepts involving cell cycle arrest and DNA damage response (including RAD51, ATM, and BUB1). The performance of the refined radiation sensitivity signature was cross-validated in the training set as able to significantly distinguish patients who would recur locoregionally in the future on univariate and multivariate analysis. The radiation sensitivity score was then subsequently validated in an independent breast cancer dataset and was again able to accurately discriminate patients who developed locoregional recurrence from those who did not. The radiation sensitivity score was again the most significant factor in predicting local recurrence on multivariate analysis and outperformed all currently used clinicopathologic features associated with local recurrence.

Experiments conducted during development of embodiments of the present invention derived a human breast cancer-specific radiation sensitivity signature with biologic relevance from preclinical studies and validated the signature for prediction of locoregional recurrence in an independent clinical data set. This signature outperforms all previously developed signatures and clinicopathologic features associated with local recurrence. The signature is not correlated to the intrinsic subtypes of human breast cancer and therefore represents "value added" information over traditional breast cancer subtyping. By identifying patients with tumors refractory to standard radiation this signature allows for personalization of radiotherapy, particularly in patients for whom treatment intensification is needed.

### A. Patient Cohorts.

Two publicly available clinical cohorts were utilized. A multi-institutional training cohort of 343 patients from the Netherlands and France with early stage breast cancer treated with breastconserving surgery with post-op radiation (Servant, N., et al., Clin Cancer Res, 2012. 18(6): p. 1704-15.), and a validation cohort of 295 patients from the Netherlands with early stage breast cancer treated surgically with radiation as indicated (van de Vijver, M.J., et al., N Engl J Med, 2002. 347(25): p. 1999-2009.).

### B. RNA isolation and Quantitative RT-PCR (Q-RT-PCR).

Total RNA was isolated using the RNeasy RNA isolation kit (QIAGEN). Quantitative RT-PCR assays of transcripts were carried out using gene-specific double fluorescence-labeled probes in an ABI PRISM 7500 Sequence Detector (Applied Biosystem). The PCR reaction mixture contained 300nM each of the forward and reverse primers, 100nM probe, 0.025 units/µl of Taq Polymerase (Invitrogen), 125µM each of dNTP, 5mM MgC12, and IX Taq Polymerase buffer. Cycling conditions were 95°C for 30 seconds, followed by 40 cycles at 95°C for 5 seconds and 60°C for 30 seconds 6-Carboxy fluorescein (FAM) was used as the 5' fluorescent reporter and black hole quencher (BHQ1) was used at the 3'end quencher. All reactions were performed using triplicate RNA samples. Standard curves for the quantification of each transcript were generated using a serially diluted solution of synthetic templates. Results were reported as average expression ± standard error of the mean.

### 1. Cell culture and cell lines

ZR75-1, MDA-MB-231, MDA-MB-468, MDA-MB-453, HCC 1954, and HCC 1937 breast cancer cell lines were grown in RPMI 1640 (Invitrogen, Carlsbad, CA) supplemented with 10% FBS (Invitrogen) in a 5% CO₂ cell culture incubator. MDA-MB-436 and Sk-BR3 breast cancer cell lines were grown in DMEM (Invitrogen, Carlsbad, CA) supplemented with 10% FBS (Invitrogen) in a 5% CO₂ cell culture incubator. SUM-149 breast cancer cell lines were grown in Ham's F12 medium (Gibco) supplemented with 5% FBS (Invitrogen), 1000 µg/ul insulin, and 500 µl of hydrocortisone (2 mg/µl) in a 5% CO₂ cell culture incubator. MRC-5 cells were maintained in Eagle minimum essential medium supplemented with 10% FBS. MCF-10A cell were maintained in MEGM medium with Single Quots added (Lonza). All cell lines were purchased from ATCC or Deutsche Sammlung von Mikroorganismens und Zellkulturen GmbH (DSMZ). All cultures were also maintained with 50 units/ml of Penicillin/streptomycin (Invitrogen). Experiments were conducted on exponentially growing cells.

### 2. Clonogenic survival assays

Exponentially growing cells were treated with drugs/radiation at doses as indicated and then replated at cloning densities chosen to demonstrate the greatest dynamic range in the survival assays. Cells were grown for up to 14 days and then fixed and stained with methanol-acetic acid and crystal violet, respectively, and scored for colonies of 50 cells or more. Drug cytotoxicity was calculated as the ratio of surviving drug-treated cells relative to untreated control cells. Radiation survival data from drug-treated cells were corrected for drug cytotoxicity, as previously described (Han, S., et al., Neoplasia, 2013. 15(10): p. 1207-17.). Cell survival curves were fitted using the linear-quadratic equation, and the mean inactivation dose calculated according to the method of Fertil and colleagues (Fertil, B., et al., Radiat Res, 1984. 99(1): p. 73-84.). The radiation enhancement ratio (EnhR) was calculated as the ratio of the mean inactivation dose under control conditions divided by the mean inactivation dose under drug-treated conditions

### 3. Immunoblotting

Cell pellets were lysed and immunoblotted (Brenner, J.C., et al., Cancer Cell, 2011. 19(5): p. 664-78.). Proteins were detected with anti-TACC1, RND3, DTL (Cell Signaling, Cat #9532), and anti-GAPDH antibody (Cell Signaling, Cat #2118)

### 4. Irradiation

Irradiation was carried out using a Philips RT250 (Kimtron Medical) at a dose rate of ~2 Gy/min in the University of Michigan Comprehensive Cancer Center Experimental Irradiation Core. Dosimetry was carried out using an ionization chamber connected to an electrometer system that is directly traceable to a National Institute of Standards and Technology calibration.

### C. Statistical Analyses

### 1. Microarrays:

Normalized expression data for the cell lines was downloaded from the Wellcome Trust Sanger Institute. Normalized expression data for the training cohort was downloaded from the Gene Expression Omnibus (GSE30682). Normalized expression data was obtained for the validation cohort. All expression data was log transformed, and median centered and scaled in order to make the disparate platforms comparable.

### 2. Discovery in vitro:

Clonogenic survival was performed after radiation on 16 cell lines as described above. The surviving fraction after 2 Gy of radiation (SF-2Gy) was calculated for all 16 cell lines. Spearman's correlation was performed correlating the SF-2Gy to the radiation naive expression of every gene on the microarrays. A fold change was calculated for every gene between the 25th and 75th percentiles of expression. Genes were selected for initial inclusion in the model if the Spearman's correlationp < 0.05 or with a fold change > 2. Unsupervised hierarchical clustering, was performed on the expression of these genes in the BC cell lines.

### 3. Training and validation:

The set of genes selected from the cell lines described above was then used to train a Random Forest model from the training cohort. Only genes present on both the microarray platforms in the training and validation cohorts were included as not all the genes were present given the differing microarray platforms. The prognostic value of each gene by itself was calculated using a student's T-test comparing expression in samples from patients who developed local recurrence in the future, and patients who didn't. P-value cutoffs from p < 1 to p < 0.05 were used to select genes, using progressively stricter increments of 0.05. Performance of each subset of genes was determined using out-of-bag (OOB) error rate, an internal cross-validation mechanism of Random Forest models, and the subset that had the best performance was selected as the refined gene signature. The OOB predictions of the final refined signature were used to evaluate the cross-validated performance in the training cohort. This signature was then locked and used to classify the validation cohort without further modification. Enrichment of biological concepts was performed on the genes in the final signature using ConceptGen (conceptgen. ncibi. org/).

### 4. Statistical analysis:

Receiver operating characteristic (ROC) curves were generated based on the raw Random Forest voting frequency. Classification of samples into high and low risk groups used the majority vote. The performance of these predictions for clinical outcomes in the training and validation cohorts was evaluated using Fisher's exact test. Kaplan Meier curves were generated and compared using a Log-rank test. Univariable and Multivariable analysis was performed using Cox regression. R software packages (r-project.org) were used for all data and statistical analysis.

### D. Development of a radiation response signature.

To develop a molecular biomarker signature for radiation response, a strategy was employed to investigate the intrinsic radiosensitivity of 16 breast cancer cell lines chosen to represent the heterogeneity found within human breast cancer (SEE FIG. 6). For the purposes of signature development, 16 breast cancer cell lines were choosen and the long-term intrinsic radiation sensitivity of these cell lines was characterized by performing clonogenic survival assays after varying doses of ionizing radiation. This selection included 5 luminal, 4 basal A, 4 basal B, and 3 HER2/*neu* amplified cell lines as defined by previous gene expression profiling studies (Hu, X., et al., Mol Cancer Res, 2009. 7(4): p. 511-22.; Neve, R.M., et al., Cancer Cell, 2006. 10(6): p. 515-27.). The surviving fraction after 2 Gy (the typical daily fractions dose size used clinically for breast cancer treatment) of ionizing radiation was calculated based on these data and represents the intrinsic radiosensitivity of these human breast cancer cell lines (SEE FIG. 7). There was strong correlation between the SF-2Gy value and other metrics of radiation sensitivity (DBar, Gy (1%), AUC) for clonogenic survival and thus SF-2Gy values were used for further signature development. The clonogenic survival assays identified a previously unappreciated broad range of intrinsic radiosensitivity across the 16 breast cancer cell lines with SF-2Gy values ranging from 77-17%.

### E. Intrinsic breast cancer radiosensitivity is independent of subtype.

It was then determined whether the intrinsic radiosensitivity of the 16 breast cancer cell lines was correlated to the previously defined intrinsic subtypes of human breast cancer, as significant correlation would indicate that radiation sensitivity is closely related to the intrinsic subtypes and thus obfuscate the benefit of further radiation signature development. Correlation of the intrinsic radiosensitivity of breast cancer cell lines to the intrinsic subtype of human breast cancer revealed no significant association between the radiosensitivity of the cell lines and their respective intrinsic subtype (SEE FIG. 7B).

### F. Radiation sensitivity signature development.

To develop the radiation response signature, gene expression data from the 16 breast cancer cell lines was used and the correlation coefficient was calculated of the basal gene expression values with the radiation sensitivity metric (SF-2Gy) on a gene-by-gene basis. Gene expression values that were either positively or negatively correlated with radiation sensitivity (as measured by clonogenic survival assays) with a Spearman's correlationp-value < 0.05 with a fold change > 2 fold were retained within the signature (representative gene shown in FIG. 7C). This analysis identified 147 genes (67 genes positively correlated, 80 genes negatively correlated) whose expression was significantly correlated with SF-2Gy (SEE FIG. 7D and Table 5).

**Table 5. Genes positively and negatively associated with clonogenic survival in breast cancer cell lines**

| **Genes positively and negatively associated with clonogenic survival in breast cancer cell lines** | | | |
|---|---|---|---|
| **67 Genes Positively Correlated with Radioresistance** | | | |
| **Gene Symbol** | **Gene Name** | **Correlation Coeff.** | ***P*-value** |
| MED17 | mediator complex subunit 17 | 0.83 | <0.01 |
| TACC1 | transforming, acidic coiled-coil containing protein 1 | 0.78 | <0.01 |
| ATM | Serine-protein kinase ATM | 0.69 | <0.01 |
| CEP57 | centrosomal protein 57kDa | 0.68 | 0.01 |
| ARMCX2 | armadillo repeat containing, X-linked 2 | 0.67 | 0.00 |
| ANKRD49 | ankyrin repeat domain 49 | 0.67 | 0.01 |
| TXNRD1 | mitochondrial translational release factor 1 | 0.66 | 0.01 |
| MTRF1 | thioredoxin reductase 1 | 0.66 | 0.01 |
| ZNF259 | zinc finger protein 259 | 0.65 | 0.01 |
| SQSTM1 | sequestosome 1 | 0.64 | 0.01 |
| PDGFC | platelet derived growth factor C | 0.63 | 0.01 |
| RNF160 | ring finger protein 160 | 0.63 | 0.01 |
| SGMS1 | sphingomyelin synthase 1 | 0.62 | 0.01 |
| OSTM1 | osteopetrosis associated transmembrane protein 1 | 0.61 | 0.01 |
| NFIL3 | nuclear factor, interleukin 3 regulated | 0.61 | 0.01 |
| PRKCA | protein kinase C, alpha | 0.61 | 0.01 |
| PBX3 | pre-B-cell leukemia homeobox 3 | 0.61 | 0.01 |
| KIAA0802 | KIAA0802 | 0.60 | 0.01 |
| BIRC2 | baculoviral IAP repeat-containing 2 | 0.60 | 0.02 |
| LAMP2 | lysosomal-associated membrane protein 2 guanine nucleotide binding protein inhibiting activity peptide | 0.59 | 0.02 |
| GNAI1 | 1 | 0.59 | 0.02 |
| TMX4 | thioredoxin-related transmembrane protein 4 | 0.59 | 0.02 |
| RND3 | Rho family GTPase 3 | 0.59 | 0.02 |
| TAF1D | TATA box binding protein (TBP)-associated factor | 0.59 | 0.02 |
| ZNF532 | similar to zinc finger protein 347; zinc finger protein 532 | 0.58 | 0.02 |
| DNAJB4 | DnaJ (Hsp40) homolog, subfamily B, member 4 | 0.58 | 0.02 |
| VAMP7 | vesicle-associated membrane protein 7 | 0.58 | 0.02 |
| CEP170 | centrosomal protein 170kDa | 0.57 | 0.02 |
| SARS | seryl-tRNA synthetase | 0.57 | 0.02 |
| TNIK | TRAF2 and NCK interacting kinase | 0.57 | 0.02 |
| RPRD1A | regulation of nuclear pre-mRNA domain containing 1A | 0.56 | 0.02 |
| ZNF177 | zinc finger protein 177 | 0.56 | 0.02 |
| VLDLR | very low density lipoprotein receptor | 0.56 | 0.03 |
| ITPR1 | inositol 1,4,5-triphosphate receptor, type 1 | 0.55 | 0.03 |
| MSRA | methionine sulfoxide reductase A | 0.55 | 0.03 |
| STAC | prostaglandin E receptor 4 (subtype EP4) | 0.55 | 0.03 |
| PTGER4 | SH3 and cysteine rich domain | 0.55 | 0.03 |
| TOR1B | torsin family 1, member B (torsin B) | 0.54 | 0.03 |
| SERPINE2 | serpin peptidase inhibitor member 2 | 0.54 | 0.03 |
| BIN1 | bridging integrator 1 | 0.54 | 0.03 |
| TFCP2 | transcription factor CP2 | 0.54 | 0.03 |
| SDHD | breast cancer anti-estrogen resistance 3 | 0.54 | 0.03 |
| BCAR3 | similar to succinate dehydrogenase complex, subunit D | 0.54 | 0.03 |
| OSMR | oncostatin M receptor | 0.54 | 0.03 |
| DNAJC24 | DnaJ (Hsp40) homolog, subfamily C, member 24 | 0.54 | 0.03 |
| EMP3 | epithelial membrane protein 3 | 0.54 | 0.03 |
| GALC | galactosylceramidase | 0.54 | 0.03 |
| TSC22D1 | chromodomain protein, Y-like | 0.53 | 0.04 |
| CDYL | TSC22 domain family, member 1 | 0.53 | 0.04 |
| WDR47 | WD repeat domain 47 | 0.53 | 0.03 |
| EIF3M | adhesion molecule with Ig-like domain 2 | 0.53 | 0.04 |
| AMIGO2 | eukaryotic translation initiation factor 3, subunit M | 0.53 | 0.04 |
| SLC39A14 | cysteine-rich, angiogenic inducer, 61 | 0.52 | 0.04 |
| CYR61 | solute carrier family 39 (zinc transporter), member 14 | 0.52 | 0.04 |
| C6orf120 | chromosome 6 open reading frame 120 | 0.52 | 0.04 |
| FZD2 | frizzled homolog 2 (Drosophila) | 0.52 | 0.04 |
| PXMP4 | peroxisomal membrane protein 4, 24kDa | 0.52 | 0.04 |
| ZNF204P | zinc finger protein 204 pseudogene | 0.52 | 0.04 |
| C14orf139 | chromosome 14 open reading frame 139 | 0.52 | 0.04 |
| SMAP1 | small ArfGAP 1 | 0.52 | 0.04 |
| STC1 | stanniocalcin 1 | 0.52 | 0.04 |
| SGCE | phosphoserine aminotransferase 1 | 0.51 | 0.04 |
| PSAT1 | sarcoglycan, epsilon | 0.51 | 0.04 |
| STK17A | serine/threonine kinase 17a | 0.51 | 0.05 |
| MAMLD1 | mastermind-like domain containing 1 BTB and CNC homology 1 basic leucine zipper transcription | 0.50 | 0.05 |
| BACH1 | factor 1 | 0.50 | 0.05 |
| CCDC90B | coiled-coil domain containing 90B | 0.50 | 0.05 |

| **80 Genes Negatively Correlated with Radioresistance** | | | |
|---|---|---|---|
| **Gene Symbo** | **Gene Name Correlation** | **Coeff.** | ***P*-value** |
| EMP2 | epithelial membrane protein 2 | -0.78 | <0.01 |
| C4orfl9 | chromosome 4 open reading frame 19 | -0.77 | <0.01 |
| DTL | denticleless homolog | -0.77 | <0.01 |
| FBP1 | fructose-1,6-bisphosphatase 1 | -0.74 | <0.01 |
| DSP | desmoplakin | -0.71 | <0.01 |
| C19orf21 | chromosome 19 open reading frame 21 | -0.68 | <0.01 |
| COBL | cordon-bleu homolog | -0.67 | <0.01 |
| APOD | apolipoprotein D | -0.66 | 0.01 |
| BOLA2 | bolA homolog 2 (E. coli); bolA homolog 2B | -0.66 | 0.01 |
| CPE | carboxypeptidase E | -0.65 | 0.01 |
| PYCRL | pyrroline-5-carboxylate reductase-like | -0.64 | 0.01 |
| LAD1 | ladinin 1 | -0.62 | 0.01 |
| MGLL | monoglyceride lipase phosphatidylinositol-specific phospholipase C domain | -0.61 | 0.01 |
| PLCXD1 | containing 1 | -0.61 | 0.01 |
| MCM2 | minichromosome maintenance complex component 2 | -0.61 | 0.01 |
| MTSS1 | metastasis suppressor 1 | -0.61 | 0.01 |
| PDZD2 | PDZ domain containing 2 | -0.61 | 0.01 |
| TSPAN13 | tetraspanin 13 | -0.61 | 0.01 |
| BLM | Bloom syndrome, RecQ helicase-like | -0.60 | 0.02 |
| BTG2 | BTG family, member 2 | -0.60 | 0.02 |
| CDKN1A | cyclin-dependent kinase inhibitor 1A (p21, Cipl) | -0.60 | 0.02 |
| RABGAP1L | RAB GTPase activating protein 1-like | -0.60 | 0.02 |
| ABHD11 | abhydrolase domain containing 11 | -0.59 | 0.02 |
| POLE2 | polymerase (DNA directed), epsilon 2 (p59 subunit) | -0.59 | 0.02 |
| FXYD3 | FXYD domain containing ion transport regulator 3 | -0.59 | 0.02 |
| HOXC13 | homeobox C13 | -0.59 | 0.02 |
| TDRD1 | tudor domain containing 1 | -0.59 | 0.02 |
| CNKSR1 | connector enhancer of kinase suppressor of Ras 1 | -0.59 | 0.02 |
| GLB1L2 | galactosidase, beta 1-like 2 | -0.58 | 0.02 |
| MAPK3 | mitogen-activated protein kinase 3 | -0.58 | 0.02 |
| HOOK1 | hook homolog 1 (Drosophila) | -0.57 | 0.02 |
| AGR2 | anterior gradient homolog 2 | -0.57 | 0.02 |
| LPAR2 | lysophosphatidic acid receptor 2 | -0.56 | 0.02 |
| PDCD4 | programmed cell death 4 | -0.56 | 0.02 |
| PKP1 | plakophilin 1 | -0.56 | 0.02 |
| SRD5A1 | steroid-5-alpha-reductase, alpha polypeptide 1 | -0.56 | 0.02 |
| S100P | S100 calcium binding protein P | -0.56 | 0.03 |
| PRC1 | protein regulator of cytokinesis 1 | -0.56 | 0.03 |
| PLEKHA1 | pleckstrin homology domain containing, family A member 1 | -0.56 | 0.03 |
| BUB1B | budding uninhibited by benzimidazoles 1 homolog beta | -0.55 | 0.03 |
| SIVA1 | SIVA1, apoptosis-inducing factor | -0.55 | 0.03 |
| HSPA2 | heat shock 70kDa protein 2 | -0.55 | 0.03 |
| RBPMS | RNA binding protein with multiple splicing | -0.55 | 0.03 |
| LMCD1 | LIM and cysteine-rich domains 1 | -0.55 | 0.03 |
| TJP2 | tight junction protein 2 (zona occludens 2) | -0.55 | 0.03 |
| DNMT3B | DNA (cytosine-5-)-methyltransferase 3 beta | -0.54 | 0.03 |
| TK1 | thymidine kinase 1, soluble | -0.54 | 0.03 |
| MKI67 | antigen identified by monoclonal antibody Ki-67 | -0.54 | 0.03 |
| DCI | dodecenoyl-Coenzyme A delta isomerase | -0.54 | 0.03 |
| RAB15 | RAB15, member RAS onocogene family | -0.54 | 0.03 |
| MCM10 | minichromosome maintenance complex component 10 | -0.53 | 0.04 |
| CDT1 | chromatin licensing and DNA replication factor 1 | -0.53 | 0.04 |
| HNMT | histamine N-methyltransferase | -0.53 | 0.04 |
| MYH14 | myosin, heavy chain 14 | -0.53 | 0.04 |
| RAD51 | RAD51 homolog (RecA homolog, E. coli) | -0.53 | 0.04 |
| TRIM14 | tripartite motif-containing 14 | -0.52 | 0.04 |
| SKP2 | S-phase kinase-associated protein 2 (p45) | -0.52 | 0.04 |
| ISG20 | interferon stimulated exonuclease gene 20kDa | -0.52 | 0.04 |
| C22orf9 | chromosome 22 open reading frame 9 | -0.52 | 0.04 |
| CALHM2 | calcium homeostasis modulator 2 | -0.52 | 0.04 |
| HELLS | helicase, lymphoid-specific | -0.52 | 0.04 |
| LOC100272216 | hypothetical LOC100272216 | -0.52 | 0.04 |
| KRT86 | keratin 86 | -0.52 | 0.04 |
| MSLN | mesothelin | -0.52 | 0.04 |
| SLC44A1 | solute carrier family 44, member 1 | -0.52 | 0.04 |
| CORO2A | coronin, actin binding protein, 2A | -0.51 | 0.04 |
| MYO5C | myosin VC | -0.51 | 0.04 |
| S100A13 | S100 calcium binding protein A13 | -0.51 | 0.04 |
| BATF | basic leucine zipper transcription factor, ATF-like phospholysine phosphohistidine inorganic pyrophosph. | -0.51 | 0.04 |
| LHPP | phosphatase | -0.51 | 0.04 |
| GINS2 | GINS complex subunit 2 (Psf2 homolog) | -0.51 | 0.05 |
| MCM5 | minichromosome maintenance complex component 5 | -0.51 | 0.05 |
| PBX1 | pre-B-cell leukemia homeobox 1 | -0.51 | 0.05 |
| RAB25 | RAB25, member RAS oncogene family | -0.51 | 0.05 |
| BEX4 | brain expressed, X-linked 4 | -0.50 | 0.05 |
| PAQR4 | progestin and adipoQ receptor family member IV | -0.50 | 0.05 |
| SPC25 | SPC25, NDC80 kinetochore complex component, homolog | -0.50 | 0.05 |
| C13orf34 | chromosome 13 open reading frame 34 | -0.50 | 0.05 |
| ASF1B | ASF1 anti-silencing function 1 homolog B | -0.50 | 0.05 |
| FA2H | fatty acid 2-hydroxylase | -0.50 | 0.05 |

An unsupervised hierarchical clustering was performed to evaluate the strength of association between gene expression and radiosensitivity. Unsupervised hierarchical clustering identifies two distinct groups of cell lines and appropriately clusters the radioresistant cell lines (SF-2Gy >45%) distinct from the radiosensitive cell lines (SF-2Gy <45%) (SEE FIG. 8).

### F. Signature development identifies novel radiation sensitivity-related genes.

Having identified genes whose expression was significantly correlated with radiation sensitivity as measured by clonogenic survival capacity, a number of these genes were validated as being differentially expressed at both the RNA and protein level (SEE FIG. 9), and experiments were performed to determine whether any of the identified associated genes played a role in the radioresistance phenotype. To determine whether the expression-to-radiosensitivity correlation played a meaningful role in the radiation resistant phenotype, the effect of gene expression knockdown in the radioresistant cell lines was interogated to determine the radiosensitizing effect of single gene manipulation. *TACC1, RND3,* and *DTL* were identified as being involved in the radiosensitivity of human breast cancer cell lines (SEE FIG. 9C). Expression of TACC1 and RND3 was increased in the cell lines that were found to be the most radioresistant in clonogenic survival assays. Knockdown of *TACC1* and *RND3* in the radioresistant cell line MDA-MB-231 using siRNA approaches identified significant radiosensitization with single gene knockdown in these cell lines (radiation enhancement ratios of 1.31-1.43 to 1.22-1.32, respectively; SEE FIG. 9D).

### G. Refinement of the radiation sensitivity signature.

Having utilized a correlative approach to identify genes whose expression was significantly correlated with radiation sensitivity *in vitro,* experiments were conducted to further refine the signature by identifying genes contributing most significantly to the signature's performance and incorporating the outcomes data from a clinical cohort. To that end a human breast tumor dataset was identified for which gene expression levels were known and for which there was long-term follow-up, including local recurrence information provided (Servant, N., et al., Clin Cancer Res, 2012. 18(6): p. 1704-15.). This training dataset included 343 patients with greater than 10-year follow-up for whom long-term locoregional recurrence events was known. The majority of the patients had early-stage disease with small primary tumors (≤pT2) and all were managed surgically with breast conserving therapy followed by radiation. The majority of the patients (63%, 215 patients) had pathologically lymph node-negative disease, while a minority (37%, 128 patients) were lymph node-positive. The majority (68%) of these patients did not receive systemic chemotherapy and the locoregional recurrence rate in this population was relatively high (25%). Given the need for an increased number of locoregional recurrence events to train our signature, this cohort intentionally was selected with this bias to improve the training of our signature for future validation in independent datasets.

This training cohort was used to train and refine the radiation sensitivity signature to 51 genes (Table 6). In an effort to define the underlying mechanisms of radiation sensitivity in breast cancer, biological concepts were analyzed that were enriched in these 51 genes. It was found that biological concepts related to the cell cycle, DNA damage, and DNA were significantly enriched (SEE FIG. 10).

**Table 6. Genes positively and negatively associated with clonogenic survival in breast cancer cell lines**

| | | |
|---|---|---|
| **Genes positively and negatively associated with clonogenic survival in breast cancer cell lines** | | |
| **23 Genes Positively Correlated with Radioresistance** | | |

| **Gene symbol** | **Gene Name** | **Correlation Coeff.** |
|---|---|---|
| ATM | ataxia telangiectasia mutated | 0.69 |
| MTRF1 | mitochondrial translational release factor 1 | 0.66 |
| ZNF259 | zinc finger protein 259 | 0.65 |
| ZNF294 | zinc finger protein 294 | 0.63 |
| PBX3 | pre-B-cell leukemia homeobox 3 | 0.61 |
| NFIL3 | nuclear factor, interleukin 3 regulated | 0.61 |
| PRKCA | protein kinase C, alpha | 0.61 |
| LAMP2 | lysosomal-associated membrane protein 2 | 0.59 |
| SYBL1 | synaptobrevin-like 1 | 0.58 |
| DNAJB4 | DnaJ (Hsp40) homolog, subfamily B, member 4 | 0.58 |
| SARS | seryl-tRNA synthetase | 0.57 |
| ITPR1 | inositol 1,4,5-triphosphate receptor, type 1 | 0.55 |
| MSRA | methionine sulfoxide reductase A | 0.55 |
| BIN1 | bridging integrator 1 | 0.54 |
| SERPINE2 | serpin peptidase inhibitor, clade E member 2 | 0.54 |
| EMP3 | epithelial membrane protein 3 | 0.54 |
| GALC | galactosylceramidase | 0.54 |
| BCAR3 | breast cancer anti-estrogen resistance 3 | 0.54 |
| OSMR | oncostatin M receptor | 0.54 |
| SDHD | succinate dehydrogenase complex, subunit D | 0.54 |
| FZD2 | frizzled homolog 2 (Drosophila) | 0.52 |
| SLC39A14 | solute carrier family 39 (zinc transporter), member 14 | 0.52 |
| PXMP4 | peroxisomal membrane protein 4, 24kDa | 0.52 |

| **28 Genes Negatively Correlated with Radioresistance** | | |
|---|---|---|
| **Gene Symbol** | **Gene Name** | **Correlation Coeff.** |
| RAB25 | RAB25, member RAS oncogene family | -0.5 |
| SPC25 | SPC25, NDC80 kinetochore complex component, homolog | -0.5 |
| ASF1B | ASF1 anti-silencing function 1 homolog B | -0.5 |
| BATF | basic leucine zipper transcription factor, ATF-like | -0.51 |
| MYO5C | myosin VC | -0.51 |
| GINS2 | GINS complex subunit 2 (Psf2 homolog) | -0.51 |
| KRT86 | keratin 86 | -0.52 |
| HELLS | helicase, lymphoid-specific | -0.52 |
| RAD51 | RAD51 homolog (RecA homolog, E. coli) | -0.52 |
| TK1 | thymidine kinase 1, soluble | -0.53 |
| MCM10 | minichromosome maintenance complex component 10 | -0.53 |
| CDT1 | chromatin licensing and DNA replication factor 1 | -0.53 |
| MKI67 | antigen identified by monoclonal antibody Ki-67 | -0.54 |
| DNMT3B | DNA (cytosine-5-)-methyltransferase 3 beta | -0.54 |
| RAB15 | RAB15, member RAS onocogene family | -0.54 |
| BUB1B | BUB1 budding uninhibited by benzimidazoles 1 homolog | -0.55 |
| RBPMS | RNA binding protein with multiple splicing | -0.55 |
| PRC1 | protein regulator of cytokinesis 1 | -0.56 |
| PDCD4 | programmed cell death 4 | -0.56 |
| HOOK1 | hook homolog 1 (Drosophila) | -0.57 |
| HOXC13 | homeobox C13 | -0.59 |
| BLM | Bloom syndrome | -0.6 |
| BTG2 | BTG family, member 2 | -0.6 |
| TSPAN13 | tetraspanin 13 | -0.61 |
| CPE | carboxypeptidase E | -0.65 |
| COBL | cordon-bleu homolog | -0.67 |
| DTL | denticleless homolog | -0.77 |
| C4orf19 | chromosome 4 open reading frame 19 | -0.77 |

The performance of the radiation sensitivity signature in the training cohort was perfect with an ROC AUC of 0.99 (SEE FIG 11A,C). For internal cross-validation a Random Forest out-of-bag (OOB) prediction model was used. This technique accurately distinguished patients with recurrence after radiation from those without recurrence with a log-rankp-value <10⁻⁶ and hazard ratio (HR) = 2.5 (SEE FIG. 11B,D). On univariable and multivariable Cox regression analysis the radiosensitivity score predictions were the single most predictive clinical or pathologic variable, with a log-rank p-value of <0.0001 and HR = 2.8 in multivariable analysis (SEE FIG. 11E,F).

### H. Independent validation of the radiation sensitivity signature.

An independent human breast tumor dataset was identified with gene expression and long term clinical outcomes that included locoregional recurrence in which to validate our radiation sensitivity signature (van de Vijver, M.J., et al., N Engl J Med, 2002. 347(25): p. 1999-2009.). The validation cohort included 295 patients with a minimum of 5-year follow-up with locoregional recurrence and overall survival endpoints. Similar to the training set, all the patients had early-stage disease (≤pT2) and the majority received adjuvant radiation therapy and did not receive systemic chemotherapy as standard of care. The locoregional recurrence rate in this population was lower than the training cohort, and included only 27 events (9%) which more closely reflect the event rates in current series.

Evaluation of the performance of the radiation sensitivity signature in the validation dataset demonstrated an AUC comparable to the out-of-bag cross-validation at 0.74 and was significantly better than any other clinical or pathologic parameter (FIG. 12A). The radiation sensitivity signature predicted those who would develop recurrence remarkably well, misclassifying only four events giving it a sensitivity of 85% and a negative predictive value (NPV) of 97% with a Log-rankp-value <0.001 (HR=6.1) for locoregional recurrence (SEE FIG. 12B). Once again, on univariable and multivariable analysis, the radiation sensitivity signature score is better than all other prognostic clinical or pathologic variables (SEE FIG 12C,D). It was found that the radiation sensitivity signature score was also highly prognostic for overall survival, with a Log-rank p-value <0.0001 with a HR=2.82. This finding is robust and the radiation sensitivity score remains prognostic on multivariate analysis for overall with a *p*-value <0.001 and the largest HR (2.2) of all the variables. Thus, not only was a radiation sensitivity signature identified that is highly sensitive and specific for local recurrence, but also is prognostic for overall survival in an independent breast cancer dataset.

### I. Radiation sensitivity signature outperforms previously described radiation related signatures.

Previous groups have also attempted to develop a predictive radiation signatures. These signatures, though not derived in a breast cancer specific manner, have been applied to breast cancer datasets to assess the ability to risk-stratify breast cancer patients after radiation treatment (Eschrich, S.A., et al. Clin Cancer Res. 18(18): p. 5134-43.). The performance of a signature described herein against this previously reported signature in the training and validation cohort was analyzed; the breast cancer-specific signature described herein outperforms other signatures in both of these datasets (SEE FIGS. 13-14).

### REFERENCES

Jemal, A., et al., Cancer statistics, 2009. CA Cancer J Clin, 2009. 59(4): p. 225-49.
Early Breast Cancer Trialists' Collaborative, G., et al., Effect of radiotherapy after breastconserving surgery on 10-year recurrence and 15-year breast cancer death: meta-analysis of individual patient data for 10,801 women in 17 randomised trials. Lancet, 2011. 378(9804): p. 1707-16.
Sorlie, T., et al., Gene expression patterns of breast carcinomas distinguish tumor subclasses with clinical implications. Proc Natl Acad Sci USA, 2001. 98(19): p. 10869-74.
Sorlie, T., et al., Repeated observation of breast tumor subtypes in independent gene expression data sets. Proc Natl Acad Sci USA, 2003. 100(14): p. 8418-23.
Cancer Genome Atlas, N., Comprehensive molecular portraits of human breast tumours. Nature, 2012. 490(7418): p. 61-70.
Kyndi, M., et al., Estrogen receptor, progesterone receptor, HER-2, and response to postmastectomy radiotherapy in high-risk breast cancer: the Danish Breast Cancer Cooperative Group. J Clin Oncol, 2008. 26(9): p. 1419-26.
Eschrich, S.A., et al., Validation of a radiosensitivity molecular signature in breast cancer. Clin Cancer Res. 18(18): p. 5134-43.
Naderi, A., et al., A gene-expression signature to predict survival in breast cancer across independent data sets. Oncogene, 2007. 26(10): p. 1507-16.
Weichselbaum, R.R., et al., An interferon-related gene signature for DNA damage resistance is a predictive marker for chemotherapy and radiation for breast cancer. Proc Natl Acad Sci U S A, 2008. 105(47): p. 18490-5.
Torres-Roca, J.F., et al., Prediction of radiation sensitivity using a gene expression classifier. Cancer Res, 2005. 65(16): p. 7169-76.
Servant, N., et al., Search for a gene expression signature of breast cancer local recurrence in young women. Clin Cancer Res, 2012. 18(6): p. 1704-15.
Hu, X., et al., Genetic alterations and oncogenic pathways associated with breast cancer subtypes. Mol Cancer Res, 2009. 7(4): p. 511-22.
Neve, R.M., et al., A collection of breast cancer cell lines for the study of functionally distinct cancer subtypes. Cancer Cell, 2006. 10(6): p. 515-27.
van de Vijver, M.J., et al., A gene-expression signature as a predictor of survival in breast cancer. N Engl J Med, 2002. 347(25): p. 1999-2009.
Clarke, M., et al., Effects of radiotherapy and of differences in the extent of surgery for early breast cancer on local recurrence and 15-year survival: an overview of the randomised trials. Lancet, 2005. 366(9503): p. 2087-106.
Kandoth, C., et al., Mutational landscape and significance across 12 major cancer types. Nature, 2013. 502(7471): p. 333-9.
Lawrence, M.S., et al., Discovery and saturation analysis of cancer genes across 21 tumour types. Nature, 2014. 505(7484): p. 495-501.
Wistuba, II, et al., Comparison of features of human breast cancer cell lines and their corresponding tumors. Clin Cancer Res, 1998. 4(12): p. 2931-8.
Boeckman, H.J., K.S. Trego, and J.J. Turchi, Cisplatin sensitizes cancer cells to ionizing radiation via inhibition of nonhomologous end joining. Mol Cancer Res, 2005. 3(5): p. 277-85.
Lawrence, T.S., A.W. Blackstock, and C. McGinn, The mechanism of action of radiosensitization of conventional chemotherapeutic agents. Semin Radiat Oncol, 2003. 13(1): p. 13-21.
Gonzalez de Castro, D., et al., Personalized cancer medicine: molecular diagnostics, predictive biomarkers, and drug resistance. Clin Pharmacol Ther, 2013. 93(3): p. 252-9.
Simon, R.M., S. Paik, and D.F. Hayes, Use of archived specimens in evaluation of prognostic and predictive biomarkers. J Natl Cancer Inst, 2009. 101(21): p. 1446-52.
Han, S., et al., Targeted radiosensitization of ETS fusion-positive prostate cancer through PARP1 inhibition. Neoplasia, 2013. 15(10): p. 1207-17.
Fertil, B., et al., Mean inactivation dose: a useful concept for intercomparison of human cell survival curves. Radiat Res, 1984. 99(1): p. 73-84.
Brenner, J.C., et al., Mechanistic rationale for inhibition of poly(ADP-ribose) polymerase in ETS gene fusion-positive prostate cancer. Cancer Cell, 2011. 19(5): p. 664-78.

## Claims

1. A kit for assessing radiosensitivity of acell, comprising reagents selected from the group consisting of probes, primers, antibodies and aptamers for detecting expression of aplurality genes indicative of radiosensitivity and/or radioresistance, wherein
i) said genes are the 51 genes in Table 6; and
ii) said kit does not include reagents for analyzing other markers than said 51 genes in said Table 6.

2. The kit of claim 1, wherein said kit further comprises buffer.

3. A method for assessing the radiosensitivity of apopulation of breast cancer cells within asubject, said method comprising:
(a) receiving asample obtained from the population of cells;
(b) quantitating the level of expression of the 51 genes in Table 6 indicative of radiosensitivity in said sample; and
(c) quantitating the level of expression of the 51 genes in Table 6 indicative of radioresistance in said sample,
wherein said method is further defined by the following features:
i) said gene expression is detected by measuring mRNA levels or protein levels; and/or
ii) the expression of said plurality genes indicative of radiosensitivity and/or radioresistance are differentially weighted to determine the radiosensitivity of the cell;
or
iii) said method further comprises: generating areport related to the radiosensitivity of the population of cells.

4. A method of determining atreatment course for asubject suffering from breast cancer comprising:
(a) assessing the radiosensitivity of the cancer by the method of claim 3; and
(b) selecting asuitable treatment course based on the radiosensitivity of the cancer.

## Patentansprüche

1. Kit zur Einschätzung der Radiosensitivität einer Zelle, umfassend Reagenzien, die aus der Gruppe ausgewählt sind, die aus Sonden, Primern, Antikörpern und Aptameren zum Nachweis der Expression einer Vielzahl von Genen besteht, die auf die Radiosensitivität und/oder Radioresistenz hinweisen, wobei
i) es sich bei den Genen um die 51 Gene in Tabelle 6 handelt; und
ii) der Kit keine Reagenzien zum Analysieren anderer Marker als der 51 Gene in Tabelle 6 enthält.

2. Kit nach Anspruch 1, wobei der Kit des Weiteren Puffer enthält.

3. Verfahren zur Einschätzung der Radiosensitivität einer Population von Brustkrebszellen in einem Subjekt, wobei das Verfahren umfasst:
(a) Erhalt einer aus der Zellpopulation gewonnenen Probe;
(b) Quantifizierung des Expressionsniveaus der 51 Gene in Tabelle 6, die auf die Radiosensitivität in dieser Probe hinweisen; und
(c) Quantifizierung des Expressionsniveaus der 51 Gene in Tabelle 6, die auf Radioresistenz in der Probe hinweisen,
wobei das Verfahren des Weiteren durch die folgenden Merkmale definiert ist:
i) die Genexpression wird durch Messung der mRNA-Spiegel oder der Proteinspiegel ermittelt; und/oder
ii) die Expression der Vielzahl von Genen, die auf Radiosensitivität und/oder Radioresistenz hinweisen, werden unterschiedlich gewichtet, um die Radiosensitivität der Zelle zu bestimmen;
oder
iii) das Verfahren umfasst des Weiteren: Erzeugen eines Berichts in Bezug auf die Radiosensitivität der Zellpopulation.

4. Verfahren zur Bestimmung eines Behandlungsplans für ein an Brustkrebs erkranktes Subjekt, umfassend:
(a) Einschätzen der Radiosensitivität des Krebses nach dem Verfahren von Anspruch 3; und
(b) Auswählen eines geeigneten Behandlungsplans auf der Grundlage der Radiosensitivität des Krebses.

## Revendications

1. Trousse servant à évaluer la radiosensibilité d'une cellule, comprenant des réactifs choisis dans le groupe constitué par des sondes, des amorces, des anticorps et des aptamères pour détecter l'expression d'une pluralité de gènes indicateurs de la radiosensibilité et/ou de la radiorésistance, dans laquelle
i) lesdits gènes sont les 51 gènes dans le tableau 6 ; et
ii) ladite trousse ne comporte pas de réactifs destinés à analyser d'autres marqueurs que lesdits 51 gènes dans le tableau 6.

2. Trousse de la revendication 1, ladite trousse comprenant en outre un tampon.

3. Procédé servant à évaluer la radiosensibilité d'une population de cellules de cancer du sein chez un sujet, ledit procédé comprenant :
(a) la réception d'un échantillon obtenu à partir de la population de cellules ;
(b) la quantification du niveau d'expression des 51 gènes dans le tableau 6 indicateurs de la radiosensibilité dans ledit échantillon ; et
(c) la quantification du niveau d'expression des 51 gènes dans le tableau 6 indicateurs de la radiorésistance dans ledit échantillon,
ledit procédé étant en outre défini par les caractéristiques suivantes :
i) ladite expression génique est détectée par mesure de niveaux d'ARNm ou de niveaux de protéines ; et/ou
ii) l'expression de ladite pluralité de gènes indicateurs de la radiosensibilité et/ou de la radiorésistance est pondérée de façon différentielle pour déterminer la radiosensibilité de la cellule ;
ou
iii) ledit procédé comprend en outre : la génération d'un rapport relatif à la radiosensibilité de la population de cellules.

4. Procédé de détermination d'un traitement pour un sujet souffrant d'un cancer du sein comprenant :
(a) l'évaluation de la radiosensibilité du cancer par le procédé de la revendication 3 ; et
(b) la sélection d'un traitement approprié sur la base de la radiosensibilité du cancer.
